(19) Europäisches Patentamt
European Patent Office
Office européen des brevets

(11) Publication number: 0 167 920
A2

# (12) EUROPEAN PATENT APPLICATION

(21) Application number: 85107844.4

(22) Date of filing: 25.06.85

(51) Int. Cl.⁴: C 07 D 243/18
C 07 D 403/06, A 61 K 31/55

(30) Priority: 26.06.84 US 624852
10.06.85 US 741973

(43) Date of publication of application:
15.01.86 Bulletin 86/3

(84) Designated Contracting States:
AT BE CH DE FR GB IT LI LU NL SE

(71) Applicant: MERCK & CO. INC.
126, East Lincoln Avenue P.O. Box 2000
Rahway New Jersey 07065(US)

(72) Inventor: Freidinger, Roger M.
2185 Rebecca Drive
Hatfield PA 19440(US)

(72) Inventor: Bock, Mark G.
1603 Leon Drive
Hatfield PA 19440(US)

(72) Inventor: Evans, Ben E.
501 Perkiomen Avenue
Lansdale PA 19446(US)

(74) Representative: Blum, Rudolf Emil Ernst et al,
c/o E. Blum & Co Patentanwälte Vorderberg 11
CH-8044 Zürich(CH)

(54) **Benzodiazepine derivatives and pharmaceutical compositions containing them.**

(57) Benzodiazepines of the formula:

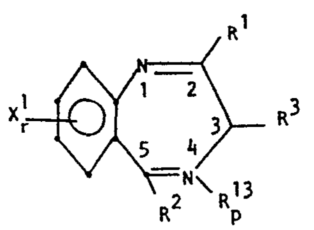

are disclosed which are antagonists of cholecystokinin (CCK).

EP 0 167 920 A2

## TITLE OF THE INVENTION

Benzodiazepine derivatives and pharmaceutical compositions containing them.

## CROSS-REFERENCE

The benzodiazepinones (Formula IV) which are the starting materials for the compounds of Formula I are described in a patent application filed on even date herewith entitled "Benzodiazepine Analogs" in the names of Freidinger, Evans, and Bock, Merck & Co., Inc. attorney docket number 17119IB, which is incorporated herein by reference.

## BACKGROUND OF THE INVENTION

Cholecystokinin (CCK) is a neuropeptide composed of thirty-three aminoacids in its originally isolated form. See: Mutt and Jorpes, Biochem. J. 125 678 (1971). Also occurring in circulation are 39, 12 and 8 amino acid forms. The carboxyl terminal octapeptide (CCK-8) is the minimum fully active sequence. Gastrin occurs in 34, 17, and 14 amino acid forms in circulation and is related to CCK by identity of the C-terminal pentapeptides Gly-Trp-Met-Asp-Phe-$NH_2$. Gastrin and CCK exist

in both gastrointestinal tissue and the central nervous system. V. Mutt, Gastrointestinal Hormones, G. B. J. Glass, Ed., Raven Press, N.Y., p. 169 and G. Nisson, ibid, p. 127. CCK is believed to play an important role in appetite regulation and CCK may be a physiological satiety hormone. G. P. Smith, Eating and Its Disorders, A. J. Stunkard and E. Stellar, Eds, Raven Press, New York, 1984, p. 67.

Among additional effects of CCK are stimulation of colonic motility, stimulation of gall bladder contraction, stimulation of pancreatic enzyme secretion, and inhibition of gastric emptying. CCK reportedly co-exists with dopamine in certain mid-brain neurons and thus may also play a role in the functioning of dopaminergic systems in the brain, as well as serving as a neurotransmitter in its own right. See: A. J. Prange et al., "Peptides in the Central Nervous System", Ann. Repts. Med. Chem. 17 31, 33 (1982) and references cited therein; J. A. Williams, Biomed. Res. 3 107 (1982); and J. E. Morley, Life Sci. 30, 479, (1982).

The primary role of gastrin appears to be stimulation of secretion of water and electrolytes from the stomach and it is therefore involved in control of gastric acid secretion.

CCK antagonists are useful in the treatment and prevention of CCK-related disorders of the gastrointestinal, central nervous and appetite regulatory systems of animals, especially humans. Three distinct chemical classes of CCK receptor antagonists have been reported. One class comprises derivatives of cyclic nucleotides; detailed structure-function

studies have demonstrated that of the various members of this class, dibutyryl cyclic GMP is the most potent. See; N. Barlos et al., Am. J. Physiol., 242, G 161 (1982) and P. Robberecht et al., Mol., Pharmacol., 17, 268 (1980). The second class comprises peptide antagonists which are C-terminal fragments and analogs of CCK. Recent structure-function studies have shown that both shorter C-terminal fragments of CCK (Boc-Met-Asp-Phe-$NH_2$, Met-Asp-Phe-$NH_2$) as well as longer CCK fragments Cbz-Tyr($SO_3H$)-Met-Gly-Trp-Met-Asp-$NH_2$) can function as CCK antagonists. See: R. T. Jensen et al., Biochem. Biophys. Acta., 757, 250 (1983) and M. Spanarkel et al., J. Biol. Chem., 258, 6746 (1983). The third class of CCK receptor antagonists comprises the amino acid derivatives; proglumide, a derivative of glutaramic acid, and the N-acyl tryptophans including para-chlorobenzoyl-L-tryptophan (benzotript). See W. F. Hahne et al., Proc. Natl. Acad. Sci. U.S.A., 78, 6304 (1981) and R. T. Jensen et al., Biochem. Biophys. Acta., 761, 269 (1983). All of these compounds are relatively weak antagonists of CCK ($IC_{50}$: $10^{-4}$-$10^{-6}$M; generally, $10^{-4}$M but down to $10^{-6}$M in the case of peptides). The peptide antagonists have substantial stability and absorption problems.

Gastrin antogonists are useful in the treatment and prevention of gastrin-related disorders of the gastrointestinal systems of humans and animals such as ulcers, Zollinger-Ellison syndrome, antral G cell hyperplasia and other conditions in which reduced gastrin activity is of therapeutic value. There are no effective receptor antagonists of the in

*vivo* effects of gastrin. J. S. Morley, *Gut Pept. Ulcer Proc.*, Hiroshima Symp. 2nd, 1983, page 1. Very weak *in vitro* antagonists such as proglumide and certain peptides have been reported, J. Martinez, *J. Med. Chem.*, *27*, 1597 (1984).

The benzodiazepine (BZD) structure class has been widely exploited as therapeutic agents especially as central nervous system (CNS) drugs. These compounds exhibit strong binding to "benzodiazepine receptors" *in vitro*, but have not been reported to bind to CCK or gastrin receptors. The large majority of reported BZD's do not contain substituents attached to the 3-position of the seven-membered ring. It is well known in the art that 3-substituents result in decreasing CNS activity, especially as these substituents increase in size. It has been demonstrated that the preferred stereochemistry at position 3 for CNS activity is *S*, which would correspond to an L-amino acid such as L-tryptophan. The compounds of Formula I are distinguished from BZD's of the prior art especially by the presence of 3-substituents. The formula I compounds bind strongly to CCK receptors, but only weakly to BZD receptors, especially with increasing size of the substituent. The preferred stereochemistry of Formula I compounds is opposite to that of prior art BZD's.

## SUMMARY OF THE INVENTION

It has now been found that compounds of Formula I are antagonists of cholecystokinin (CCK). These CCK antagonists are useful in the treatment and prevention of CCK-related disorders of the

gastrointestinal, central nervous and appetite regulatory systems of mammals, especially humans. The compounds of Formula I are also gastrin antagonists. They are useful in the treatment and prevention of gastrointestinal ulcers, Zollinger-Ellison syndrome, antral G cell hyperplasia, and other conditions in which reduced gastrin activity is of therapeutic value.

## DETAILED DESCRIPTION OF THE INVENTION

The compounds of this invention are those of Formula I:

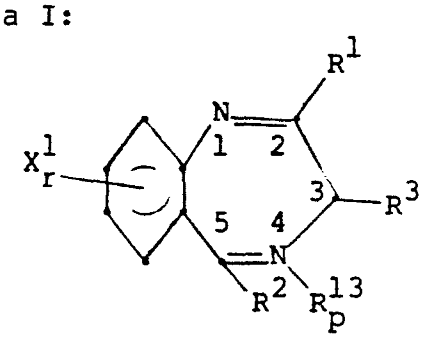

I

wherein

$R^1$ is $-NR^{16}R^{17}$, $-SR^{11}$, or $-OR^{11}$;

$R^2$ is H, loweralkyl, substituted or unsubstituted phenyl (wherein the substituents may be 1 or 2 of halo, loweralkyl, loweralkoxy, lower-alkylthio, carboxyl, carboxyloweralkyl, nitro, $-CF_3$, $O\overset{O}{\overset{\|}{C}}R^4$ or hydroxy), or $-(CH_2)_mCOOR^6$;

$R^3$ is $-(CH_2)_nR^7$, $-(CH_2)_n\overset{OH}{\overset{|}{C}}HR^7$, $-(CH_2)_nX^9\overset{O}{\overset{\|}{C}}\underset{NHCOOR^{14}}{\underset{|}{C}H}CH_2R^7$,

$-(CH_2)_nC(=O)R^7$, $-(CH_2)_nNR^{18}(CH_2)_qR^7$,

$-(CH_2)_nNR^{18}CH[(CH_2)_qR^7]COOR^6$, $-(CH_2)_nX^9C(=O)(CH_2)_qR^7$,

$-(CH_2)_nX^9C(=O)(CH_2)_qX^9_a$— 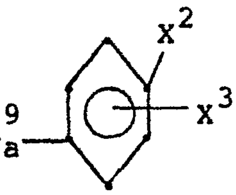 ,

$-(CH_2)_nNR^{18}SO_2(CH_2)_qR^7$, or

$-(CH_2)_n-X^9-C(=O)-X^9_a-(CH_2)_n-R^7$;

$R^4$ and $R^5$ are, when separate, independently H, loweralkyl, or cycloloweralkyl or when joined form, with the N, a heterocycle $-N(CH_2)_{n'}$ wherein n' is 2-6, and preferably 4-5;

$R^6$ is H, loweralkyl, cycloloweralkyl, substituted or unsubstituted phenyl, or substituted or unsubstituted phenylloweralkyl (wherein the substituents may be 1 or 2 of halo, loweralkyl, loweralkoxy, nitro, or $CF_3$);

$R^7$ is H, α- or β-naphthyl, substituted or unsubstituted phenyl (wherein the substituents may be 1 to 2 of halo, $-NO_2$, -OH, $-NR^4R^5$, loweralkyl, or loweralkoxy, $CF_3$, S-loweralkyl, cyano, phenyl, acetylamino, acetoxy,

$SCF_3$, C≡CH, $CH_2SCF_3$, $OCHF_2$, SH, S-phenyl, or $PO_3H$);

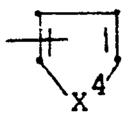 , 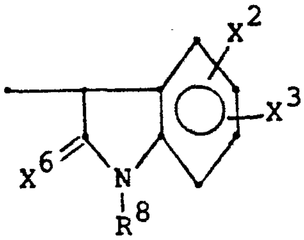 , 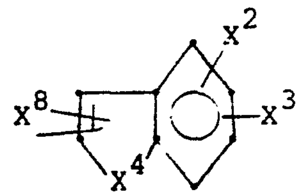 ,

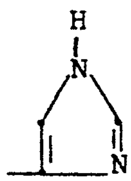 , 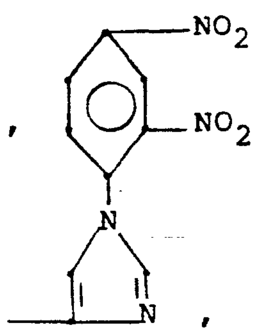 ,

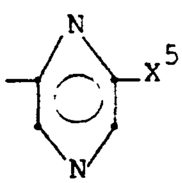 , 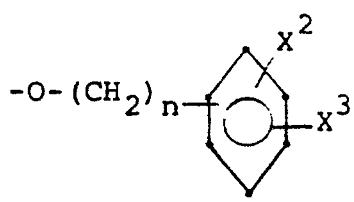 ,

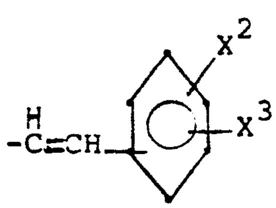 , 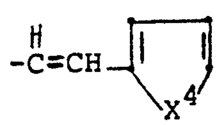 , 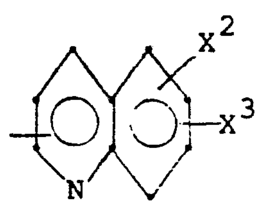 ,

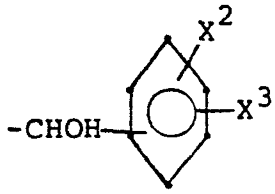 , or 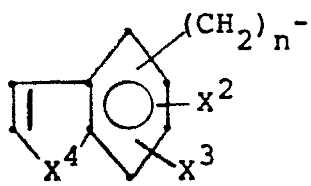

(with the provisos that q is not 0 or 1 in $-(CH_2)_nNH(CH_2)_qR^7$ and that q is not 0 in

$R^7$
|
$(CH_2)_q$
|
$-(CH_2)_nNR^{18}CHCOOR^6$ when $R^7$ is $-O-(CH_2)_n$ 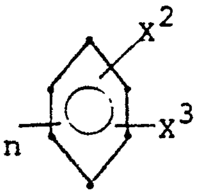)

$R^8$ is H, loweralkyl, cycloloweralkyl, $-(CH_2)_mCONH_2$, $-(CH_2)_mCOOR^6$, $-(CH_2)_n$-cycloloweralkyl,

$-(CH_2)_mNR^4R^5$ , $-(CH_2)_m$ 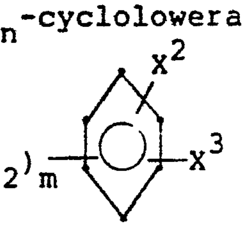 ,

$-(CH_2)_nCO(CH_2)_q$ 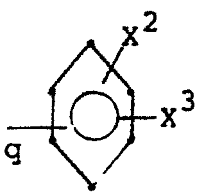 , or $-COCHNHCOOR^{11}$;
$CH_2R^{12}$

$R^{11}$ is loweralkyl or cycloloweralkyl;

$R^{12}$ is loweralkyl, cycloloweralkyl, or straight chain or branched hydroxyloweralkyl;

$R^{13}$ is O;

$R^{14}$ is loweralkyl or phenylloweralkyl;

$R^{16}$ and $R^{17}$ are, when separate, independently H, loweralkyl, 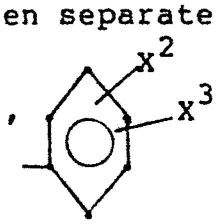, $-(CH_2)_mCOOR^6$, or -CN;

or may be joined to form a heterocycle, with N of the formula $-N\ (CH_2)_{n'}$, where n' is

2-6, preferably 4-5 (e.g., 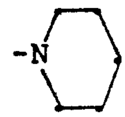, 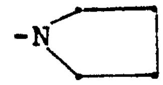)

$R^{18}$ is H, loweralkyl, or acyl;

m is 1-4;

n is 0-4;

p is 0 or 1;

q is 0-4;

r is 1 or 2;

$X^1$ is H, $-NO_2$, $CF_3$, CN, OH, loweralkyl, halo, loweralkylthio, loweralkoxy, $-(CH_2)_nCOOR^6$, $-NR^4R^5$, or $O\overset{O}{\overset{\parallel}{C}}-R^4$;

$X^2$ and $X^3$ are independently H, -OH, $-NO_2$, halo, loweralkylthio, loweralkyl, loweralkoxy, or $O\overset{O}{\overset{\parallel}{C}}R^4$;

$X^4$ is S, O, $CH_2$ or $NR^8$;

$X^5$ is H, $CF_3$, CN, $COOR^6$, $NO_2$, or halo;

$X^6$ is O or HH;

$X^8$ is H or loweralkyl;

$X^9$ and $X_a^9$ are independently $NR^{18}$, O;

and the pharmaceutically acceptable salts thereof.

In the compounds of Formula I, the preferred stereochemistry relates to D-tryptophan, where $C^2$ and $N^4$ of Formula I correspond to the carbonyl carbon and α-amino nitrogen of D-tryptophan and $R^3$ occupies the position of the indolylmethyl side chain.

As used herein, the definition of each expression, e.g. m, n, p, loweralkyl, etc., when it occurs more than once in any structure, is intended to be independent of its definition elsewhere in the same structure.

As used herein, halo is F, Cl, Br or I; loweralkyl is 1-4 carbon straight or branched chain alkyl and includes methyl, ethyl, propyl, isopropyl, butyl, isobutyl, and t-butyl; in loweralkoxy and loweralkylthio, the alkyl portion is loweralkyl as previously defined; cycloloweralkyl is cycloalkyl of 3-5 carbons; and acyl is formyl, acetyl, propionyl, or butyryl.

The pharmaceutically acceptable salts of the compounds of Formulas I include the conventional non-toxic salts or the quarternary ammonium salts of the compounds of Formula I formed, e.g., from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like.

The pharmaceutically acceptable salts of the present invention can be synthesized from the compounds of Formula I which contain a basic or acidic moiety by conventional chemical methods. Generally, the salts are prepared by reacting the free base or acid with stoichiometric amounts or with an excess of the desired salt forming inorganic or organic acid or base in a suitable solvent or various combinations of solvents.

The pharmaceutically acceptable salts of the acid of Formula I are also readily prepared by conventional procedures such as treating an acid of Formula I with an appropriate amount of a base, such as an alkali or alkaline earth metal hydroxide e.g. sodium, potassium, lithium, calcium, or magnesium, or an organic base such as an amine, e.g., dibenzylethylenediamine, trimethylamine, piperidine, pyrrolidine, benzylamine and the like, or a quaternary ammonium hydroxide such as tetramethylammonium hydroxide and the like.

An embodiment of this invention is the preparation of compounds of Formula I.

Another embodiment is the use of the compounds of Formula I for the treatment and the prevention of disorders of the gastrointestinal, central nervous, and appetite regulatory systems of mammals, especially of man. Specifically, the Formula I compounds are useful in treatment and prevention of disorders of gastric acid secretion, gastrointestinal motility, pancreatic secretions, and dopaminergic functions. The compounds of Formula I are especially useful in the prevention and treatment of irritable bowel syndrome.

A further embodiment is a composition comprising an effective amount of a compound of Formula I and a pharmaceutically acceptable carrier.

The ability of the compounds of Formula I to antagonize CCK and gastrin makes these compounds useful as pharmaceutical agents. These compounds will be especially useful in the treatment and prevention of disease states wherein CCK or gastrin

may be involved, for example, gastrointestinal disorders such as irritable bowel syndrome, ulcers, excess pancreatic or gastric secretion, acute pancreatitis, motility disorders, pain (potentiation of opiate analgesia), central nervous system disorders caused by CCK's interaction with dopamine such as neuroleptic disorders, tardive dyskinesia, Parkinson's disease, psychosis or Gilles de la Tourette Syndrome, disorders of appetite regulatory systems, Zollinger-Ellison syndrome, and antral G cell hyperplasia.

The compounds of Formula I or pharmaceutically acceptable salts thereof, can be administered to a human subject either alone, or preferably, in combination with pharmaceutically acceptable carriers or diluents, in a pharmaceutical composition, according to standard pharmaceutical practice. The compounds can be administered orally or parenterally. Parenteral administration includes intravenous, intramuscular, intraperitoneal, subcutaneous and topical administration.

For oral use of an antagonist of CCK or gastrin of this invention, the selected compound can be administered, for example, in the form of tablets or capsules, or as an aqueous solution or suspension. In the case of tablets for oral use, carriers which are commonly used include lactose and corn starch, and lubricating agents, such as magnesium stearate, are commonly added. For oral administration in capsule form, useful diluents are lactose and dried corn starch. When aqueous suspensions are required for oral use, the active

ingredient is combined with emulsifying and suspending agents. If desired, certain sweetening and/or flavoring agents can be added. For intramuscular, intraperitoneal, subcutaneous and intravenous use, sterile solutions of the active ingredient are usually prepared, and the pH of the solutions should be suitably adjusted and buffered. For intravenous use, the total concentration of solutes should be controlled to render the preparation isotonic.

When a compound of Formula I or a salt thereof is used as an antagonist of CCK or gastrin in a human subject, the daily dosage will normally be determined by the prescribing physician. Moreover, the dosage will vary according to the age, weight, and response of the individual patient, as well as the severity of the patient's symptoms. However, in most instances, an effective daily dosage will be in the range from about 0.05 mg to about 100 mg/kg and preferably 0.5 mg to about 20 mg/kg in a single or divided doses. On the other hand, it may be necessary to use dosages outside these limits in some cases.

The compounds of Formula I are prepared according to the following scheme.

REACTION SCHEME I

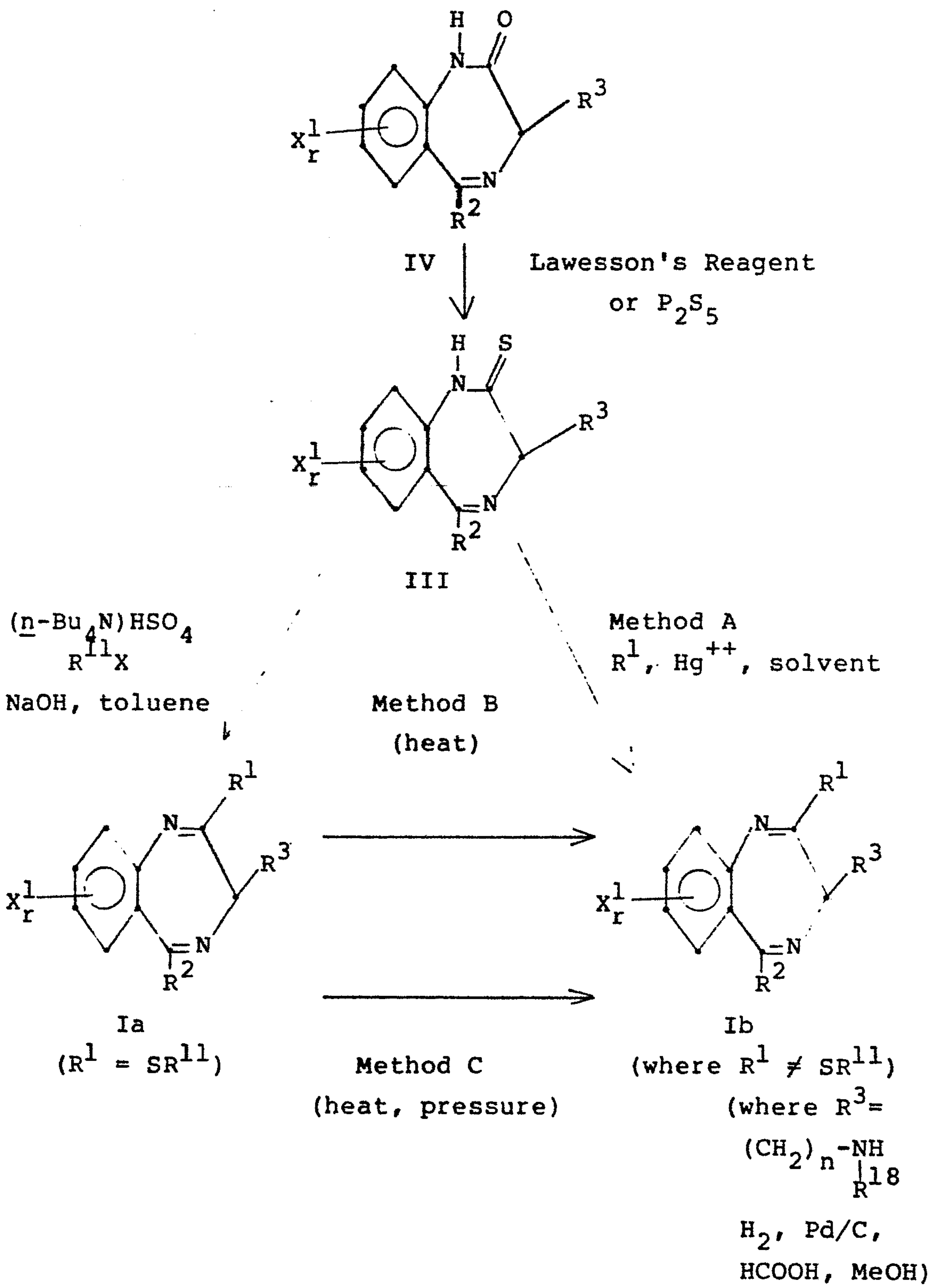

REACTION SCHEME I (Cont'd)

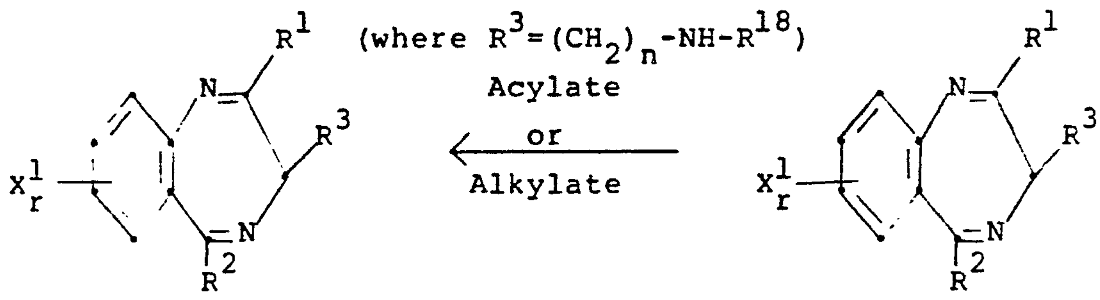


Ib Ib

Referring to Scheme I, the compounds of Formula I are prepared as follows:

1,3-Dihydro-3,5-disubstituted-1,4-benzodiazepines IV are reacted with phosphorus pentasulfide or preferably Lawesson's reagent (2,4-bis-(4-methoxyphenyl)-2,4-dithioxo-1,3,2,4-dithiadiphosphetane) in an aprotic solvent, preferably toluene at room temperature to the boiling point of the solvent, to give the corresponding thioamides III.

Treatment of the benzodiazepin-2-thiones III with an alcohol or an amine in a solvent, preferably tetrahydrofuran, in the presence of a mercury salt such as mercuric chloride or mercuric acetate affords the title compound Ib, (Method A).

Alternatively, the benzodiazepin-2-thiones III can be converted to the title compound thioiminoethers Ia with an alkylating agent, preferably a lower alkyl halide or cycloloweralkyl halide, at room temperature, under phase transfer conditions requiring aqueous base, such as an alkali earth hydroxide, an organic solvent immiscible with water, preferably toluene and a catalyst, preferably, tetra-n-butylammonium hydrogen sulfate. The thioiminoethers Ia are, in turn, transformed to the title compounds Ib by reaction with an amine or alcohol for 2-96 hours preferably 24 hours, at room temperature to the boiling point of the reagent, preferably 80°C, (Method B). Compounds of formula Ib are also accessible by heating iminoethers Ia with an amine or alcohol in a sealed pressure vessel at 80-250°C, preferably 120°, for 1-36 hours, preferably 12 hours, (Method C).

When $R^3$ in compounds of Formula IV contains an amino, keto, or alcohol moiety, this moiety should be protected during the synthesis of Ia or Ib according to methods known in the art. Removal of such protecting groups according to methods known in the art at the end the synthesis provides Ia or Ib.

For the case of $R^3$ containing a funtionalized amino group, Scheme I is performed on IV, $R^3=(CH_2)_n-\overset{R^{18}}{\overset{|}{N}}—\overset{O}{\overset{\|}{C}}-O-CH_2-Ph$. The protecting group is then removed from Ib by catalytic transfer hydrogenolysis, and the resultant amine [Ib, $R^3=(CH_2)_m-NH-R^{18}$] is derivatized appropriately, e.g. with an acid $HOOC-(CH_2)_q-R^7$ to give Ib, $R^3=(CH_2)_n-NR^{18}\underset{\underset{O}{\|}}{C}-(CH_2)_q-R^7$.

In cases where the starting materials are optically active, the chirality at $C_3$ is controlled by the synthesis. When racemic starting materials are employed, racemic mixtures are obtained. The enantiomers may be separated by resolution.

<u>In Vitro Activity of Formula I</u>

The biological activity of the compounds of Formula I have been evaluated using 1) an $^{125}I$-CCK receptor binding assay and <u>in vitro</u> isolated tissue preparations and 2) $^{125}I$-gastrin and $^{3}H$-pentagastrin binding assays.

Materials and Methods

1. CCK Receptor Binding (Pancreas)

CCK-33 was radiolabeled with $^{125}$I-Bolton Hunter reagent (2000 Ci/mmole) as described by Sankara et al. (J. Biol. Chem. 254: 9349-9351, 1979). Receptor binding was performed according to Innis and Snyder (Proc. Natl. Acad. Sci. 77: 6917-6921, 1980) with the minor modification of adding the additional protease inhibitors, phenylmethane sulfonyl fluoride and o-phenanthroline. The latter two compounds have no effect on the $^{125}$I-CCK receptor binding assay.

Male Sprague-Dawley rats (200-350g) were sacrificed by decapitation. The whole pancreas was dissected free of fat tissue and was homogenized in 20 volumes of ice-cold 50 mM, Tris HCl (pH 7.7 at 25°C) with a Brinkmann Polytron PT 10. The homogenates were centrifuged at 48,000 g for 10 min. Pellets were resuspended in Tris Buffer, centrifuged as above and resuspended in 200 volumes of binding assay buffer (50 mM Tris HCl, pH 7.7 at 25°C, 5 mM dithiothrietol, 0.1 mM bacitracin, 1.2 mM phenylmethane sulfonyl fluoride and 0.5 mM o-phenanthroline). For the binding assay, 25 µl of buffer (for total binding) or unlabeled CCK-8 sulfate to give a final concentration of 1µM (for nonspecific binding) or the compounds of Formula I (for determination of inhibition of $^{125}$I-CCK binding) and 25 µl of $^{125}$I-CCK-33 (30,000-40,000 cpm) were added to 450 µl of the membrane suspensions in microfuge tubes. All assays were run in duplicate or triplicate. The reaction mixtures were incubated at

37°C for 30 minutes and centrifuged in a Beckman Microfuge (4 minutes) immediately after adding 1 ml of ice-cold incubation buffer. The supernatant was aspirated and discarded, pellets were counted with a Beckman gamma 5000. For Scatchard analysis (Ann. N.Y. Acad. Sci. 51: 660, 1949), $^{125}$I-CCK-33 was progressively diluted with increasing concentrations of CCK-33

2. CCK Receptor Binding (Brain)

CCK-33 was radiolabeled and the binding was performed according to the description for the pancreas method with modifications according to Saito et al., J. Neurochem. 37, 483-490, 1981.

Male Hartley guinea pigs (300-500g) were sacrificed by decapitation and the brains were removed and placed in ice-cold 50 mM, Tris HCl plus 7.58 g/l Trizma-7.4 (pH 7.4 at 25°C). Cerebral cortex was dissected and used as a receptor source. Each gram of fresh guinea pig brain tissue was homogenized in 10 ml of Tris Trizma buffer with a Brinkman polytron PT-10. The homogenates were centrifuged at 42,000 g for 15 min. Pellets were resuspended in Tris Buffer, centrifuged as above and resuspended in 200 volumes of binding assay buffer (10 mM N-2-hydroxethyl-piperazine-N'-2-ethane sulfonic acid (HEPES), 5 mM $MgCl_2$, 0.25 mg/ml bacitracin, 1 mM ethylene glycol-bis-(ß-aminoethyl-ether-N,N'-tetraacetic acid (EGTA), and 0.4% bovine serum albumin (BSA)). For the binding assay, 25 µl of buffer (for total binding) or unlabeled CCK-8 sulfate to give a final concentration of 1µM (for nonspecific binding)

or the compounds of Formula I (for determination of inhibition of $^{125}$I-CCK binding) and 25 µl of $^{125}$I-CCK-33 (30,000-40,000 cpm) were added to 450 µl of the membrane suspensions in microfuge tubes. All assays were run in duplicate or triplicate. The reaction mixtures were incubated at 25°C for 2 hours and centrifuged in a Beckman Microfuge (4 minutes) immediately after adding 1 ml of ice-cold incubation buffer. The supernatant was aspirated and discarded, pellets were counted with a Beckman gamma 5000.

The compounds of Formula I can be determined to be competitive antagonists of CCK according to the following assays.

3. <u>Isolated guinea pig gall bladder</u>

Male Hartley guinea pigs (400-600 g) are sacrificed by decapitation. The whole gall bladder is dissected free from adjacent tissues and cut into two equal halves. The gall bladder strips are suspended along the axis of the bile duct in a 5 ml organ bath under 1 g tension. The organ bath contains a Kreb's bicarbonate solution (NaCl 118 mM, KCl 4.75 mM, CaCl 2.54 mM, $KH_2PO_4$ 1.19 mM, Mg $SO_4$ 1.2 mM, $NaHCO_3$ 25 mM and dextrose 11 mM) maintained at 32°C and bubbled with 95% $O_2$ and 5% $CO_2$. Isometric contractions are recorded using Statham (60 g; 0.12 mm) strain gauges and a Hewlett-Packard ( 77588) recorder. The tissues are washed every 10 minutes for 1 hr to obtain equilibrium prior to the beginning of the study. CCK-8 is added cumulatively to the baths and $EC_{50}$'s determined using regression analysis. After washout

(every 10 minutes for 1 hr), the compound of Formula I is added at least 5 minutes before the addition of CCK-8 and the $EC_{50}$ of CCK-8 in the presence of the compound of Formula I similarly determined.

4. Isolated longitudinal muscle of guinea pig ileum

Longitudinal muscle strips with attached nerve plexus are prepared as described in Brit J. Pharmac. 23: ; 356-363, 1964; J. Physiol. 194: 13-33, 1969. Male Hartley guinea pigs are decapitated and the ileum removed (10 cm of the terminal ileum is discarded and the adjacent 20 cm piece used). A piece (10 cm) of the ileum is stretched on a glass pipette. Using a cotton applicator to stroke tangentially away from the mesentery attachment at one end, the longitudinal muscle is separated from the underlying circular muscle. The longitudinal muscle is then tied to a thread and by gently pulling, stripped away from the entire muscle. A piece of approximately 2 cm is suspended in 5 ml organ bath containing Krebs solution and bubbled with 95% $O_2$ and 5% $CO_2$ at 37°C under 0.5 g tension. CCK-8 is added cumulatively to the baths and $EC_{50}$ values in the presence and absence of compounds of Formula I determined as described in the gall bladder protocol (above).

5. Gastrin Antagonism

Gastrin antagonist activity of compounds of Formula I is determined using the following assay:

Gastrin Receptor Binding in Guinea Pig Gastric Glands

Preparation of guinea pig gastric mucosal glands

Guinea pig gastric mucosal glands were prepared by the procedure of Berglingh and Obrink Acta Physiol. Scand. 96: 150 (1976) with a slight modification according to Praissman *et al*. C. J. Receptor Res. 3: (1983). Gastric mucosa from guinea pigs (300-500 g body weight, male Hartley) were washed thoroughly and minced with fine scissors in standard buffer consisting of the following: 130 mM NaCl, 12 mM $NaHCO_3$, 3 mM $NaH_2PO_4$, 3 mM $Na_2HPO_4$, 3 mM $K_2HPO_4$, 2 mM $MgSO_4$, 1 mM $CaCl_2$, 5 mM glucose and 4 mM L-glutamine, 25 mM HEPES at pH 7.4. The minced tissues were washed and then incubated in a 37°C shaker bath for 40 minutes with the buffer containing 0.1% collagenase and 0.1% BSA and bubbled with 95% $O_2$ and 5% $CO_2$. The tissues were passed twice through a 5 ml glass syringe to liberate the gastric glands, and then filtered through 200 mesh nylon. The filtered glands were centrifuged at 270 g for 5 minutes and washed twice by resuspension and centrifugation.

In Vitro Results

1. Effect of The Compounds of Formula I on $^{125}$I-CCK-33 receptor binding

The preferred compounds of Formula I are those which inhibited specific $^{125}$I-CCK-33 binding in a concentration dependent manner.

Scatchard analysis of specific $^{125}$I-CCK-33 receptor binding in the absence and presence of the compounds of Formula I indicated the compound of

Formula I competitively inhibited specific $^{125}I$-CCK-33 receptor binding since it increased the $K_D$ (dissociation constant) without affecting the $B_{max}$ (maximum receptor number). A $K_i$ value (dissociation constant of inhibitor) of the compounds of Formula I was estimated.

The data of Table 1 were obtained for compounds of the following structure:

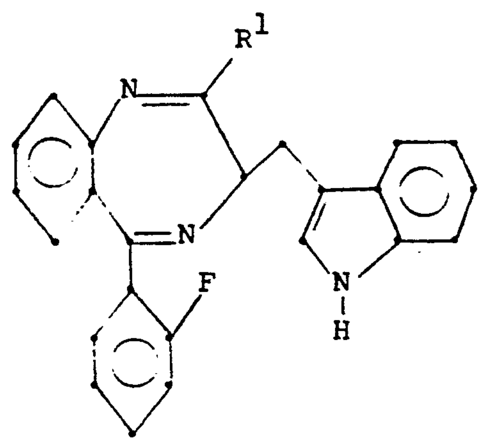

TABLE 1

Receptor Binding Results

| $R^1$ | $IC_{50}$ (μM) $^{125}I$-CCK, pancreas |
|---|---|
| PhNH | 34 |
| $CH_3S$ | 17 |
| $NH_2$ | 1.7 |
| $CH_3NH$ | 4.4 |
| $EtOCOCH_2NH$ | 23 |
| NC-NH | 2.0 |
| $CH_3CH_2CH_2NH$ | 2.7 |
| $HOOC-CH_2-NH$ | 3.6 |

Preferred compounds of Formula I are those where $R^1$ is $-NH_2$, $-NH(CH_2)_{0-2}-CH_3$, $NH-(CH_2)_{1-2}-COOH$ or -NHCN.

Other series of preferred compounds are those wherein $R^2$ is phenyl, p-chlorophenyl, o-chlorophenyl, o-fluorophenyl, 2,4-dichlorophenyl, 2,6-difluorophenyl, $-CH_2COO$-t-butyl, or $-CH_2COOEt$.

Other series of preferred compounds are those where $R^3$ is 2- or 3-indolylmethyl; NHCO-$R^7$ where $R^7$ is 2-indolyl, 2-(1-methylindolyl), 2-(5-fluoroindolyl), 2-benzofuranyl, 2-benzothienyl, 2-(3-methylindenyl), phenylethenyl, mono- or dihalophenyl, mono- or dimethyl or trifluoromethylphenyl; CHOH-1-methylindol-3-yl; NHCONH-p-chlorophenyl; CO-2-(1-methyl-indolyl); CO-3-(1-methyl-indolyl); or CO-thiophene.

It is preferred that $X^1_r$ is H, Cl, F, $CF_3$, OH or $NO_2$.

Examples of Formula I compounds are tabulated below.

TABLE 2

Compounds of the formula:

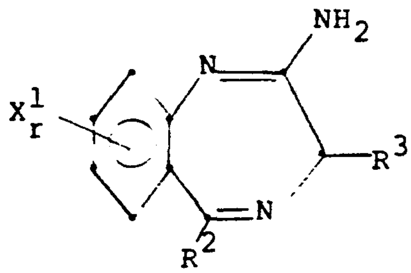

| $X^1$ | r | $R^2$ | $R^3$ |
|---|---|---|---|
| H | 1 | o-F-Ph | $-CH_2$-2-indolyl |
| H | 1 | o-F-Ph | $-CH_2$-3-indolyl |
| H | 1 | o-F-Ph | 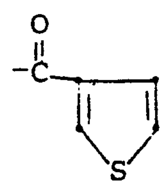 |
| H | 1 | o-F-Ph | 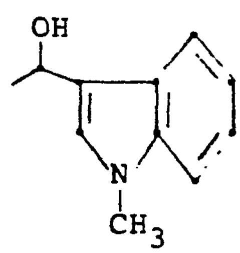 |
| H | 1 | o-F-Ph- | $NH(CH_2)_{1-3}$-3-indolyl |

TABLE 2 (cont'd)

| $X^1$ | r | $R^2$ | $R^3$ |
|---|---|---|---|
| H | 1 | o-F-Ph | $-NHCH(COOEt)-(CH_2)_2$-3-indolyl |
| H | 1 | o-F-Ph | $-CH_2NH(CH_2)_2$-3-indolyl |
| H | 1 | o-F-Ph | $-NHCO-(CH_2)_{0-2}$-2-indolyl |
| H | 1 | o-F-Ph | $-NHCO(CH_2)_{0-2}$-3-indolyl |
| H | 1 | o-F-Ph | -CO-2-indolyl |
| H | 1 | o-F-Ph | -CO-3-indolyl |
| H | 1 | o-F-Ph | NHCO-p-halo-phenyl |
| H | 1 | o-F-Ph | NHCO-m-halo-phenyl |
| H | 1 | o-F-Ph | NHCO-2-benzofuranyl |
| H | 1 | o-F-Ph | 2-(3-methylindenyl) |
| H | 1 | o-F-Ph | phenylethenyl |
| H | 1 | o-F-Ph | NHCONH-p-halophenyl |
| H | 1 | p-Cl-Ph | $-CH_2$-2-indolyl |

TABLE 2 (cont'd)

| $X^1$ | r | $R^2$ | $R^3$ |
|---|---|---|---|
| H | 1 | p-Cl-Ph | $-CH_2$-3-indolyl |
| H | 1 | p-Cl-Ph | $-NH(CH_2)_{1-3}$-3-indolyl |
| H | 1 | p-Cl-Ph | -CHOH-1-methylindol-3-yl |
| H | 1 | p-Cl-Ph | $-NHCO-(CH_2)_{0-2}$-2-indolyl |
| H | 1 | p-Cl-Ph | NHCO-p-halo-phenyl |
| H | 1 | p-Cl-Ph | NHCO-m-halo-phenyl |
| H | 1 | p-Cl-Ph | NHCO-2-benzofuranyl |
| H | 1 | p-Cl-Ph | 2-(3-methylindenyl) |
| H | 1 | p-Cl-Ph | phenylethenyl |
| H | 1 | p-Cl-Ph | NHCONH-p-halo-phenyl |
| H | 1 | phenyl | $-CH_2$-2-indolyl |
| H | 1 | phenyl | $-CH_2$-3-indolyl |
| H | 1 | phenyl | $-NHCO(CH_2)_{0-2}$-indolyl |
| H | 1 | phenyl | -CHOH-1-methylindol-3-yl |

TABLE 2 (cont'd)

| $X^1$ | r | $R^2$ | $R^3$ |
|---|---|---|---|
| H | 1 | phenyl | $-NH(CH_2)_{1-3}$-3-indolyl |
| H | 1 | phenyl | NHCO-p-halo-phenyl |
| H | 1 | phenyl | NHCO-m-halo-phenyl |
| H | 1 | phenyl | NHCO-2-benzofuranyl |
| H | 1 | phenyl | 2-(3-methylindenyl) |
| H | 1 | phenyl | phenylethenyl |
| H | 1 | phenyl | NHCONH-p-halo-phenyl |
| H | 1 | p-F-Ph | $-CH_2$-2-indolyl |
| H | 1 | p-F-Ph | $-CH_2$-3-indolyl |
| H | 1 | p-F-Ph | $-NHCO(CH_2)_{0-2}$-2-indolyl |
| H | 1 | p-F-Ph | -CHOH-1-methylindol-3-yl |
| H | 1 | p-F-Ph | $-NH(CH_2)_{1-3}$-3-indolyl |
| H | 1 | p-F-Ph | NHCO-p-halo-phenyl |
| H | 1 | p-F-Ph | NHCO-m-halo-phenyl |

TABLE 2 (cont'd)

| $X^1$ | r | $R^2$ | $R^3$ |
|---|---|---|---|
| H | 1 | p-F-Ph | NHCO-2-benzofuranyl |
| H | 1 | p-F-Ph | 2-(3-methylindenyl) |
| H | 1 | p-F-Ph | phenylethenyl |
| H | 1 | p-F-Ph | NHCONH-p-halophenyl |
| H | 1 | 2,4-di-Cl-Ph | $-CH_2$-2-indolyl |
| H | 1 | 2,4-di-Cl-Ph | $-CH_2$-3-indolyl |
| H | 1 | 2,4-di-Cl-Ph | $-NHCO(CH_2)_{0-2}$-2-indolyl |
| H | 1 | 2,4-di-Cl-Ph | -CHOH-1-methylindol-3-yl |
| H | 1 | 2,4-di-Cl-Ph | $-NH(CH_2)_{1-3}$-3-indolyl |
| H | 1 | 2,4-di-Cl-Ph | NHCO-p-halo-phenyl |
| H | 1 | 2,4-di-Cl-Ph | NHCO-m-halo-phenyl |
| H | 1 | 2,4-di-Cl-Ph | NHCO-2-benzofuranyl |
| H | 1 | 2,4-di-Cl-Ph | 2-(3-methylindenyl) |
| H | 1 | 2,4-di-Cl-Ph | phenylethenyl |

TABLE 2 (cont'd)

| $X^1$ | r | $R^2$ | $R^3$ |
|---|---|---|---|
| H | 1 | 2,4-di-Cl-Ph | NHCONH-p-halophenyl |
| H | 1 | 2,6-di-F-Ph | $-CH_2$-2-indolyl |
| H | 1 | 2,6-di-F-Ph | $-CH_2$-3-indolyl |
| H | 1 | 2,6-di-F-Ph | $-NHCO(CH_2)_{0-2}$-2-indolyl |
| H | 1 | 2,6-di-F-Ph | -CHOH-1-methylindol-3-yl |
| H | 1 | 2,6-di-F-Ph | $-NH(CH_2)_{1-3}$-3-indolyl |
| H | 1 | 2,6-di-F-Ph | NHCO-p-halo-phenyl |
| H | 1 | 2,6-di-F-Ph | NHCO-m-halo-phenyl |
| H | 1 | 2,6-di-F-Ph | NHCO-2-benzofuranyl |
| H | 1 | 2,6-di-F-Ph | 2-(3-methylindenyl) |
| H | 1 | 2,6-di-F-Ph | phenylethenyl |
| H | 1 | 2,6-di-F-Ph | NHCONH-p-halo-phenyl |
| H | 1 | $CH_2COO$-t-butyl | $-CH_2$-2-indolyl |
| H | 1 | $CH_2COO$-t-butyl | $-CH_2$-3-indolyl |

TABLE 2 (cont'd)

| $X^1$ | r | $R^2$ | $R^3$ |
|---|---|---|---|
| H | 1 | $CH_2COO$-t-butyl | $-NHCO(CH_2)_{0-2}$-indolyl |
| H | 1 | $CH_2COO$-t-butyl | -CHOH-1-methylindol-3-yl |
| H | 1 | $CH_2COO$-t-butyl | $-NH(CH_2)_{1-3}$-3-indolyl |
| H | 1 | $CH_2COO$-t-butyl | NHCO-p-halo-phenyl |
| H | 1 | $CH_2COO$-t-butyl | NHCO-m-halo-phenyl |
| H | 1 | $CH_2COO$-t-butyl | NHCO-2-benzofuranyl |
| H | 1 | $CH_2COO$-t-butyl | 2-(3-methylindenyl) |
| H | 1 | $CH_2COO$-t-butyl | phenylethenyl |
| H | 1 | $CH_2COO$-t-butyl | NHCONH-p-halophenyl |
| H | 1 | $-CH_2COOEt$ | $-CH_2$-2-indolyl |
| H | 1 | $-CH_2COOEt$ | $-CH_2$-3-indolyl |
| H | 1 | $-CH_2COOEt$ | $-NHCO(CH_2)_{0-2}$-indolyl |
| H | 1 | $-CH_2COOEt$ | -CHOH-1-methylindol-3-yl |
| H | 1 | $-CH_2COOEt$ | $-NH(CH_2)_{1-3}$-3-indolyl |

TABLE 2 (cont'd)

| $X^1$ | r | $R^2$ | $R^3$ |
|---|---|---|---|
| H | 1 | $-CH_2COOEt$ | NHCO-p-halo-phenyl |
| H | 1 | $-CH_2COOEt$ | NHCO-m-halo-phenyl |
| H | 1 | $-CH_2COOEt$ | NHCO-2-benzofuranyl |
| H | 1 | $-CH_2COOEt$ | 2-(3-methylindenyl) |
| H | 1 | $-CH_2COOEt$ | phenylethenyl |
| H | 1 | $-CH_2COOEt$ | NHCONH-p-halophenyl |
| Cl | 1 | p-Cl-Ph | $-CH_2$-2-indolyl |
| Cl | 1 | p-Cl-Ph | $-CH_2$-3-indolyl |
| Cl | 1 | p-Cl-Ph | $-NHCO(CH_2)_{0-2}$-2-indolyl |
| Cl | 1 | p-Cl-Ph | -CHOH-1-methylidol-3-yl |
| Cl | 1 | p-Cl-Ph | $-NH(CH_2)_{1-3}$-3-indolyl |
| Cl | 1 | p-Cl-Ph | NHCO-p-halo-phenyl |
| Cl | 1 | p-Cl-Ph | NHCO-m-halo-phenyl |
| Cl | 1 | p-Cl-Ph | NHCO-2-benzofuranyl |

TABLE 2 (cont'd)

| $X^1$ | r | $R^2$ | $R^3$ |
|---|---|---|---|
| Cl | 1 | p-Cl-Ph | 2-(3-methylindenyl) |
| Cl | 1 | p-Cl-Ph | phenylethenyl |
| Cl | 1 | p-Cl-Ph | NHCONH-p-halophenyl |
| Cl | 1 | Ph | $-CH_2$-2-indolyl |
| Cl | 1 | Ph | $-CH_2$-3-indolyl |
| Cl | 1 | Ph | $-NHCO(CH_2)_{0-2}$-2-indolyl |
| Cl | 1 | Ph | -CHOH-1-methylindol-3-yl |
| Cl | 1 | Ph | $-NH(CH_2)_{1-3}$-3-indolyl |
| Cl | 1 | Ph | NHCO-p-halo-phenyl |
| Cl | 1 | Ph | NHCO-m-halo-phenyl |
| Cl | 1 | Ph | NHCO-2-benzofuranyl |
| Cl | 1 | Ph | 2-(3-methylindenyl) |
| Cl | 1 | Ph | phenylethenyl |
| Cl | 1 | Ph | NHCONH-p-halophenyl |

TABLE 2 (cont'd)

| $X^1$ | r | $R^2$ | $R^3$ |
|---|---|---|---|
| Cl | 1 | o-F-Ph | $-CH_2$-2-indolyl |
| Cl | 1 | o-F-Ph | $-CH_2$-3-indolyl |
| Cl | 1 | o-F-Ph | $-NHCO(CH_2)_{0-2}$-2-indolyl |
| Cl | 1 | o-F-Ph | -CHOH-1-methylindol-3-yl |
| Cl | 1 | o-F-Ph | $-NH(CH_2)_{1-3}$-3-indolyl |
| Cl | 1 | o-F-Ph | NHCO-p-halo-phenyl |
| Cl | 1 | o-F-Ph | NHCO-m-halo-phenyl |
| Cl | 1 | o-F-Ph | NHCO-2-benzofuranyl |
| Cl | 1 | o-F-Ph | 2-(3-methylindenyl) |
| Cl | 1 | o-F-Ph | phenylethenyl |
| Cl | 1 | o-F-Ph | NHCONH-p-halophenyl |
| Cl | 1 | p-F-Ph | $-CH_2$-2-indolyl |
| Cl | 1 | p-F-Ph | $-CH_2$-3-indolyl |
| Cl | 1 | p-F-Ph | $-NHCO(CH_2)_{0-2}$-2-indolyl |

TABLE 2 (cont'd)

| $X^1$ | r | $R^2$ | $R^3$ |
|---|---|---|---|
| Cl | 1 | p-F-Ph | -CHOH-1-methylindol-3-yl |
| Cl | 1 | p-F-Ph | $-NH(CH_2)_{1-3}$-3-indolyl |
| Cl | 1 | p-F-Ph | NHCO-p-halo-phenyl |
| Cl | 1 | p-F-Ph | NHCO-m-halo-phenyl |
| Cl | 1 | p-F-Ph | NHCO-2-benzofuranyl |
| Cl | 1 | p-F-Ph | 2-(3-methylindenyl) |
| Cl | 1 | p-F-Ph | phenylethenyl |
| Cl | 1 | p-F-Ph | NHCONH-p-halophenyl |
| F | 1 | 2,4-di-Cl-Ph | $-CH_2$-2-indolyl |
| F | 1 | 2,4-di-Cl-Ph | $-CH_2$-3-indolyl |
| F | 1 | 2,4-di-Cl-Ph | $-NHCO(CH_2)_{0-2}$-2-indolyl |
| F | 1 | 2,4-di-Cl-Ph | -CHOH-1-methylindol-3-yl |
| F | 1 | 2,4-di-Cl-Ph | $-NH(CH_2)_{1-3}$-3-indolyl |
| F | 1 | 2,4-di-Cl-Ph | NHCO-p-halo-phenyl |

TABLE 2 (cont'd)

| $X^1$ | r | $R^2$ | $R^3$ |
|---|---|---|---|
| F | 1 | 2,4-di-Cl-Ph | NHCO-m-halo-phenyl |
| F | 1 | 2,4-di-Cl-Ph | NHCO-2-benzofuranyl |
| F | 1 | 2,4-di-Cl-Ph | 2-(3-methylindenyl) |
| F | 1 | 2,4-di-Cl-Ph | phenylethenyl |
| F | 1 | 2,4-di-Cl-Ph | NHCONH-p-halophenyl |
| F | 1 | 2,6-di-F-Ph | $-CH_2$-2-indolyl |
| F | 1 | 2,6-di-F-Ph | $-CH_2$-3-indolyl |
| F | 1 | 2,6-di-F-Ph | $-NHCO(CH_2)_{0-2}$-2-indolyl |
| F | 1 | 2,6-di-F-Ph | -CHOH-1-methylindol-3-yl |
| F | 1 | 2,6-di-F-Ph | $-NH(CH_2)_{1-3}$-3-indolyl |
| F | 1 | 2,6-di-F-Ph | NHCO-p-halo-phenyl |
| F | 1 | 2,6-di-F-Ph | NHCO-m-halo-phenyl |
| F | 1 | 2,6-di-F-Ph | NHCO-2-benzofuranyl |
| F | 1 | 2,6-di-F-Ph | 2-(3-methylindenyl) |

TABLE 2 (cont'd)

| $X^1$ | r | $R^2$ | $R^3$ |
|---|---|---|---|
| F | 1 | 2,6-di-F-Ph | phenylethenyl |
| F | 1 | 2,6-di-F-Ph | NHCONH-p-halo-phenyl |
| F | 1 | COO-t-butyl | $-CH_2$-2-indolyl |
| F | 1 | COO-t-butyl | $-CH_2$-3-indolyl |
| F | 1 | COO-t-butyl | $-NHCO(CH_2)_{0-2}$-2-indolyl |
| F | 1 | COO-t-butyl | -CHOH-1-methylindol-3-yl |
| F | 1 | COO-t-butyl | $-NH(CH_2)_{1-3}$-3-indolyl |
| F | 1 | COO-t-butyl | NHCO-p-halo-phenyl |
| F | 1 | COO-t-butyl | NHCO-m-halo-phenyl |
| F | 1 | COO-t-butyl | NHCO-2-benzofuranyl |
| F | 1 | COO-t-butyl | 2-(3-methylindenyl) |
| F | 1 | COO-t-butyl | phenylethenyl |
| F | 1 | COO-t-butyl | NHCONH-p-halo-phenyl |
| F | 1 | $-CH_2COOEt$ | $-CH_2$-2-indolyl |

TABLE 2 (cont'd)

| $X^1$ | r | $R^2$ | $R^3$ |
|---|---|---|---|
| F | 1 | $-CH_2COOEt$ | $-CH_2$-3-indolyl |
| F | 1 | $-CH_2COOEt$ | $-NHCO(CH_2)_{0-2}$-2-indolyl |
| F | 1 | $-CH_2COOEt$ | -CHOH-1-methylindol-3-yl |
| F | 1 | $-CH_2COOEt$ | $-NH(CH_2)_{1-3}$-3-indolyl |
| F | 1 | $-CH_2COOEt$ | NHCO-p-halo-phenyl |
| F | 1 | $-CH_2COOEt$ | NHCO-m-halo-phenyl |
| F | 1 | $-CH_2COOEt$ | NHCO-2-benzofuranyl |
| F | 1 | $-CH_2COOEt$ | 2-(3-methylindenyl) |
| F | 1 | $-CH_2COOEt$ | phenylethenyl |
| F | 1 | $-CH_2COOEt$ | NHCONH-p-halo-phenyl |
| $CF_3$ | 1 | p-Cl-Ph | $-CH_2$-2-indolyl |
| $CF_3$ | 1 | p-Cl-Ph | $-CH_2$-3-indolyl |
| $CF_3$ | 1 | p-Cl-Ph | $-NHCO(CH_2)_{0-2}$-2-indolyl |

TABLE 2 (cont'd)

| $X^1$ | r | $R^2$ | $R^3$ |
|---|---|---|---|
| $CF_3$ | 1 | p-Cl-Ph | -CHOH-1-methylindol-3-yl |
| $CF_3$ | 1 | p-Cl-Ph | $-NH(CH_2)_{1-3}$-3-indolyl |
| $CF_3$ | 1 | p-Cl-Ph | NHCO-p-halo-phenyl |
| $CF_3$ | 1 | p-Cl-Ph | NHCO-m-halo-phenyl |
| $CF_3$ | 1 | p-Cl-Ph | NHCO-2-benzofuranyl |
| $CF_3$ | 1 | p-Cl-Ph | 2-(3-methylindenyl) |
| $CF_3$ | 1 | p-Cl-Ph | phenylethenyl |
| $CF_3$ | 1 | p-Cl-Ph | NHCONH-p-halo-phenyl |
| $CF_3$ | 1 | Ph | $-CH_2$-2-indolyl |
| $CF_3$ | 1 | Ph | $-CH_2$-3-indolyl |
| $CF_3$ | 1 | Ph | $-NHCO(CH_2)_{0-2}$-2-indolyl |
| $CF_3$ | 1 | Ph | -CHOH-1-methylindol-3-yl |
| $CF_3$ | 1 | Ph | $-NH(CH_2)_{1-3}$-3-indolyl |
| $CF_3$ | 1 | Ph | NHCO-p-halo-phenyl |

TABLE 2 (cont'd)

| $X^1$ | r | $R^2$ | $R^3$ |
|---|---|---|---|
| $CF_3$ | 1 | Ph | NHCO-m-halo-phenyl |
| $CF_3$ | 1 | Ph | NHCO-2-benzofuranyl |
| $CF_3$ | 1 | Ph | 2-(3-methylindenyl) |
| $CF_3$ | 1 | Ph | phenylethenyl |
| $CF_3$ | 1 | Ph | NHCONH-p-halo-phenyl |
| $CF_3$ | 1 | o-F-Ph | $-CH_2$-2-indolyl |
| $CF_3$ | 1 | o-F-Ph | $-CH_2$-3-indolyl |
| $CF_3$ | 1 | o-F-Ph | $-NHCO(CH_2)_{0-2}$-2-indolyl |
| $CF_3$ | 1 | o-F-Ph | -CHOH-1-methylindol-3-yl |
| $CF_3$ | 1 | o-F-Ph | $-NH(CH_2)_{1-3}$-3-indolyl |
| $CF_3$ | 1 | o-F-Ph | NHCO-p-halo-phenyl |
| $CF_3$ | 1 | o-F-Ph | NHCO-m-halo-phenyl |
| $CF_3$ | 1 | o-F-Ph | NHCO-2-benzofuranyl |
| $CF_3$ | 1 | o-F-Ph | 2-(3-methylindenyl) |

TABLE 2 (cont'd)

| $X^1$ | r | $R^2$ | $R^3$ |
|---|---|---|---|
| $CF_3$ | 1 | o-F-Ph | phenylethenyl |
| $CF_3$ | 1 | o-F-Ph | NHCONH-p-halo-phenyl |
| $CF_3$ | 1 | p-F-Ph | $-CH_2$-2-indolyl |
| $CF_3$ | 1 | p-F-Ph | $-CH_2$-3-indolyl |
| $CF_3$ | 1 | p-F-Ph | $-NHCO(CH_2)_{0-2}$-2-indolyl |
| $CF_3$ | 1 | p-F-Ph | -CHOH-1-methylindol-3-yl |
| $CF_3$ | 1 | p-F-Ph | $-NH(CH_2)_{1-3}$-3-indolyl |
| $CF_3$ | 1 | p-F-Ph | NHCO-p-halo-phenyl |
| $CF_3$ | 1 | p-F-Ph | NHCO-m-halo-phenyl |
| $CF_3$ | 1 | p-F-Ph | NHCO-2-benzofuranyl |
| $CF_3$ | 1 | p-F-Ph | 2-(3-methylindenyl) |
| $CF_3$ | 1 | p-F-Ph | phenylethenyl |
| $CF_3$ | 1 | p-F-Ph | NHCONH-p-halo-phenyl |
| $NO_2$ | 1 | p-Cl-Ph | $-CH_2$-2-indolyl |

TABLE 2 (cont'd)

| $X^1$ | r | $R^2$ | $R^3$ |
|---|---|---|---|
| $NO_2$ | 1 | p-Cl-Ph | $-CH_2$-3-indolyl |
| $NO_2$ | 1 | p-Cl-Ph | $-NHCO(CH_2)_{0-2}$-2-indolyl |
| $NO_2$ | 1 | p-Cl-Ph | -CHOH-1-methylindol-3-yl |
| $NO_2$ | 1 | p-Cl-Ph | $-NH(CH_2)_{1-3}$-3-indolyl |
| $NO_2$ | 1 | p-Cl-Ph | NHCO-p-halo-phenyl |
| $NO_2$ | 1 | p-Cl-Ph | NHCO-m-halo-phenyl |
| $NO_2$ | 1 | p-Cl-Ph | NHCO-2-benzofuranyl |
| $NO_2$ | 1 | p-Cl-Ph | 2-(3-methylindenyl) |
| $NO_2$ | 1 | p-Cl-Ph | phenylethenyl |
| $NO_2$ | 1 | p-Cl-Ph | NHCONH-p-halo-phenyl |
| $NO_2$ | 1 | Ph | $-CH_2$-2-indolyl |
| $NO_2$ | 1 | Ph | $-CH_2$-3-indolyl |
| $NO_2$ | 1 | Ph | $-NHCO(CH_2)_{0-2}$-2-indolyl |
| $NO_2$ | 1 | Ph | -CHOH-1-methylindol-3-yl |

TABLE 2 (cont'd)

| $X^1$ | r | $R^2$ | $R^3$ |
|---|---|---|---|
| $NO_2$ | 1 | Ph | $-NH(CH_2)_{1-3}$-3-indolyl |
| $NO_2$ | 1 | Ph | NHCO-p-halo-phenyl |
| $NO_2$ | 1 | Ph | NHCO-m-halo-phenyl |
| $NO_2$ | 1 | Ph | NHCO-2-benzofuranyl |
| $NO_2$ | 1 | Ph | 2-(3-methylindenyl) |
| $NO_2$ | 1 | Ph | phenylethenyl |
| $NO_2$ | 1 | Ph | NHCONH-p-halo-phenyl |
| $NO_2$ | 1 | o-F-Ph | $-CH_2$-2-indolyl |
| $NO_2$ | 1 | o-F-Ph | $-CH_2$-3-indolyl |
| $NO_2$ | 1 | o-F-Ph | $-NHCO(CH_2)_{0-2}$-2-indolyl |
| $NO_2$ | 1 | o-F-Ph | -CHOH-1-methylindol-3-yl |
| $NO_2$ | 1 | o-F-Ph | $-NH(CH_2)_{1-3}$-3-indolyl |
| $NO_2$ | 1 | o-F-Ph | NHCO-p-halo-phenyl |
| $NO_2$ | 1 | o-F-Ph | NHCO-m-halo-phenyl |

TABLE 2 (cont'd)

| $X^1$ | r | $R^2$ | $R^3$ |
|---|---|---|---|
| $NO_2$ | 1 | o-F-Ph | NHCO-2-benzofuranyl |
| $NO_2$ | 1 | o-F-Ph | 2-(3-methylindenyl) |
| $NO_2$ | 1 | o-F-Ph | phenylethenyl |
| $NO_2$ | 1 | o-F-Ph | NHCONH-p-halo-phenyl |
| $NO_2$ | 1 | p-F-Ph | $-CH_2$-2-indolyl |
| $NO_2$ | 1 | p-F-Ph | $-CH_2$-3-indolyl |
| $NO_2$ | 1 | p-F-Ph | $-NHCO(CH_2)_{0-2}$-2-indolyl |
| $NO_2$ | 1 | p-F-Ph | -CHOH-1-methylindol-3-yl |
| $NO_2$ | 1 | p-F-Ph | $-NH(CH_2)_{1-3}$-3-indolyl |
| $NO_2$ | 1 | p-F-Ph | NHCO-p-halo-phenyl |
| $NO_2$ | 1 | p-F-Ph | NHCO-m-halo-phenyl |
| $NO_2$ | 1 | p-F-Ph | NHCO-2-benzofuranyl |
| $NO_2$ | 1 | p-F-Ph | 2-(3-methylindenyl) |
| $NO_2$ | 1 | p-F-Ph | phenylethenyl |

TABLE 2 (cont'd)

| $X^1$ | r | $R^2$ | $R^3$ |
|---|---|---|---|
| $NO_2$ | 1 | p-F-Ph | NHCONH-p-halo-phenyl |
| $CH_3$ | 1 | phenyl | $-CH_2$-2-indolyl |
| $CH_3$ | 1 | phenyl | $-CH_2$-3-indolyl |
| $CH_3$ | 1 | phenyl | $-NHCO(CH_2)_{0-2}$-2-indolyl |
| $CH_3$ | 1 | phenyl | -CHOH-1-methylindol-3-yl |
| $CH_3$ | 1 | phenyl | $-NH(CH_2)_{1-3}$-3-indolyl |
| $CH_3$ | 1 | phenyl | NHCO-p-halo-phenyl |
| $CH_3$ | 1 | phenyl | NHCO-m-halo-phenyl |
| $CH_3$ | 1 | phenyl | NHCO-2-benzofuranyl |
| $CH_3$ | 1 | phenyl | 2-(3-methylindenyl) |
| $CH_3$ | 1 | phenyl | phenylethenyl |
| $CH_3$ | 1 | phenyl | NHCONH-p-halo-phenyl |
| H | 1 | o-F-Ph | $-CH_2$-2-indolyl |
| H | 1 | o-F-Ph | $-CH_2$-3-indolyl |

TABLE 2 (cont'd)

| $X^1$ | r | $R^2$ | $R^3$ |
|---|---|---|---|
| H | 1 | o-F-Ph | $-NHCO(CH_2)_{0-2}$-2-indolyl |
| H | 1 | o-F-Ph | -CHOH-1-methylindol-3-yl |
| H | 1 | o-F-Ph | $-NH(CH_2)_{1-3}$-3-indolyl |
| H | 1 | o-F-Ph | NHCO-p-halo-phenyl |
| H | 1 | o-F-Ph | NHCO-m-halo-phenyl |
| H | 1 | o-F-Ph | NHCO-2-benzofuranyl |
| H | 1 | o-F-Ph | 2-(3-methylindenyl) |
| H | 1 | o-F-Ph | phenylethenyl |
| H | 1 | o-F-Ph | NHCONH-p-halo-phenyl |
| H | 1 | p-Cl-Ph | $-CH_2$-2-indolyl |
| H | 1 | p-Cl-Ph | $-CH_2$-3-indolyl |
| H | 1 | p-Cl-Ph | $-NHCO(CH_2)_{0-2}$-2-indolyl |
| H | 1 | p-Cl-Ph | -CHOH-1-methylindol-3-yl |
| H | 1 | p-Cl-Ph | $-NH(CH_2)_{1-3}$-3-indolyl |

TABLE 2 (cont'd)

| $X^1$ | r | $R^2$ | $R^3$ |
|---|---|---|---|
| H | 1 | p-Cl-Ph | NHCO-p-halo-phenyl |
| H | 1 | p-Cl-Ph | NHCO-m-halo-phenyl |
| H | 1 | p-Cl-Ph | NHCO-2-benzofuranyl |
| H | 1 | p-Cl-Ph | 2-(3-methylindenyl) |
| H | 1 | p-Cl-Ph | phenylethenyl |
| H | 1 | p-Cl-Ph | NHCONH-p-halo-phenyl |
| H | 1 | Ph | -CO-3-thiophene |
| H | 1 | o-F-Ph | -CO-3-thiophene |
| H | 1 | p-Cl-Ph | -CO-3-thiophene |
| H | 1 | phenyl | -CO-3-thiophene |
| H | 1 | p-F-Ph | -CO-3-thiophene |
| H | 1 | 2,4-di-Cl-Ph | -CO-3-thiophene |
| H | 1 | 2,6-di-F-Ph | -CO-3-thiophene |
| H | 1 | $-CH_2COO$-t-butyl | -CO-3-thiophene |

TABLE 2 (cont'd)

| $X^1$ | r | $R^2$ | $R^3$ |
|---|---|---|---|
| H | 1 | $-CH_2COOEt$ | -CO-3-thiophene |
| H | 1 | o-F-Ph | $CH_2$-2 or 3-(1-methyl indolyl) |
| H | 1 | $-CH_2COO$-t-butyl | $CH_2$-2 or 3-(1-methyl indolyl) |
| H | 1 | $-CH_2COOEt$ | $CH_2$-2 or 3-(1-methyl indolyl) |
| H | 1 | Ph | $CH_2$-2 or 3-(1-methyl indolyl) |
| H | 1 | $-CH_2COO$-t-butyl | $NHCO(CH_2)_{0-2}$-2-indolyl |
| H | 1 | $-CH_2COOEt$ | $NHCO(CH_2)_{0-2}$-2-indolyl |
| H | 1 | Ph | $NHCO(CH_2)_{0-2}$-2-indolyl |
| H | 1 | o-F-Ph | $NHCO(CH_2)_{0-2}$-2 or 3-(1-methylindolyl) |
| H | 1 | $-CH_2COO$-t-butyl | $NHCO(CH_2)_{0-2}$-2 or 3-(1-methylindolyl) |

TABLE 2 (cont'd)

| $X^1$ | r | $R^2$ | $R^3$ |
|---|---|---|---|
| H | 1 | $-CH_2COOEt$ | $NHCO(CH_2)_{0-2}$-2 or 3-(1-methylindolyl) |
| H | 1 | Ph | $NHCO(CH_2)_{0-2}$-2 or 3-(1-methylindolyl) |
| H | 1 | o-F-Ph | Boc-Trp-NH |
| H | 1 | $-CH_2COO$-t-butyl | Boc-Trp-NH |
| H | 1 | $-CH_2COOEt$ | Boc-Trp-NH |
| H | 1 | Ph | Boc-Trp-NH |
| H | 1 | o-F-Ph | Cbz-Trp-NH |
| H | 1 | $-CH_2COO$-t-butyl | Cbz-Trp-NH |
| H | 1 | $-CH_2COOEt$ | Cbz-Trp-NH |
| H | 1 | Ph | Cbz-Trp-NH |
| H | 1 | o-F-Ph | $NH(CH_2)_{1-3}$-2-indolyl |
| H | 1 | $-CH_2COO$-t-butyl | $NH(CH_2)_{1-3}$-2-indolyl |
| H | 1 | $-CH_2COOEt$ | $NH(CH_2)_{1-3}$-2-indolyl |

TABLE 2 (cont'd)

| $X^1$ | r | $R^2$ | $R^3$ |
|---|---|---|---|
| H | 1 | Ph | $NH(CH_2)_{1-3}$-2-indolyl |
| H | 1 | o-F-Ph | $NH(CH_2)_{1-3}$-2 or 3-(1-methylindolyl) |
| H | 1 | $-CH_2COO$-t-butyl | $NH(CH_2)_{1-3}$-2 or 3-(1-methylindolyl) |
| H | 1 | $-CH_2COOEt$ | $NH(CH_2)_{1-3}$-2 or 3-(1-methylindolyl) |
| H | 1 | Ph | $NH(CH_2)_{1-3}$-2 or 3-(1-methylindolyl) |
| H | 1 | o-F-Ph | CHOH-2 or 3-indolyl |
| H | 1 | $-CH_2COO$-t-butyl | CHOH-2 or 3-indolyl |
| H | 1 | $-CH_2COOEt$ | CHOH-2 or 3-indolyl |
| H | 1 | Ph | CHOH-2 or 3-indolyl |
| H | 1 | o-F-Ph | CHOH-2-(1-methylindolyl) |
| H | 1 | $-CH_2COO$-t-butyl | CHOH-2-(1-methylindolyl) |
| H | 1 | $-CH_2COOEt$ | CHOH-2-(1-methylindolyl) |

TABLE 2 (cont'd)

| $X^1$ | r | $R^2$ | $R^3$ |
|---|---|---|---|
| H | 1 | Ph | CHOH-2-(1-methylindolyl) |
| H | 1 | $-CH_2COO$-t-butyl | CO-2-thiophene |
| H | 1 | $-CH_2COOEt$ | CO-2-thiophene |
| H | 1 | Ph | CO-2-thiophene |
| H | 1 | o-F-Ph | NHCH($CH_2$)$_2$-2-indolyl<br>\|<br>COOEt |
| H | 1 | $-CH_2COO$-t-butyl | NHCH($CH_2$)$_2$-2-indolyl<br>\|<br>COOEt |
| H | 1 | $-CH_2COOEt$ | NHCH($CH_2$)$_2$-2-indolyl<br>\|<br>COOEt |
| H | 1 | Ph | NHCH($CH_2$)$_2$-2-indolyl<br>\|<br>COOEt |
| H | 1 | o-F-Ph | COOEt<br>\|<br>NHCH($CH_2$)$_2$-2 or 3-(1-methyl-indolyl) |
| H | 1 | $-CH_2COO$-t-butyl | COOEt<br>\|<br>NHCH($CH_2$)$_2$-2 or 3-(1-methyl-indolyl) |

TABLE 2 (cont'd)

| $X^1$ | r | $R^2$ | $R^3$ |
|---|---|---|---|
| H | 1 | $-CH_2COOEt$ | COOEt<br>$NHCH(CH_2)_2$-2 or 3-(1-methyl-indolyl) |
| H | 1 | Ph | COOEt<br>$NHCH(CH_2)_2$-2 or 3-(1-methyl-indolyl) |
| H | 1 | o-F-Ph | $NHCH(CH_2)_2$-2 or 3-indolyl<br>COO-t-Butyl |
| H | 1 | $-CH_2$COO-t-butyl | $NHCH(CH_2)_2$-2 or 3-indolyl<br>COO-t-Butyl |
| H | 1 | $-CH_2COOEt$ | $NHCH(CH_2)_2$-2 or 3-indolyl<br>COO-t-Butyl |
| H | 1 | Ph | $NHCH(CH_2)_2$-2 or 3-indolyl<br>COO-t-Butyl |
| H | 1 | o-F-Ph | COO-t-Butyl<br>$NHCH(CH_2)_2$-2 or 3-(1-methyl-indolyl) |
| H | 1 | $-CH_2$COO-t-butyl | COO-t-Butyl<br>$NHCH(CH_2)_2$-2 or 3-(1-methyl-indolyl) |

TABLE 2 (cont'd)

| $X^1$ | r | $R^2$ | $R^3$ |
|---|---|---|---|
| H | 1 | $-CH_2COOEt$ | COO-t-Butyl<br>\|<br>NHCH$(CH_2)_2$-2 or 3-(1-methyl-indolyl) |
| H | 1 | Ph | COO-t-Butyl<br>\|<br>NHCH$(CH_2)_2$-2 or 3-(1-methyl-indolyl) |

TABLE 3

Compounds of the formula:

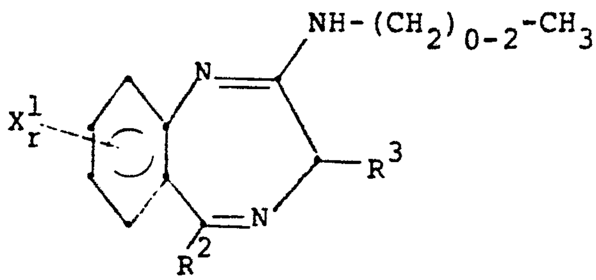

| $X^1$ | r | $R^2$ | $R^3$ |
|---|---|---|---|
| H | 1 | o-F-Ph | $-CH_2$-2-indolyl |
| H | 1 | o-F-Ph | $-CH_2$-3-indolyl |
| H | 1 | o-F-Ph | 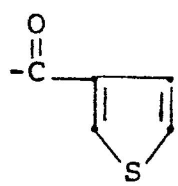 |
| H | 1 | o-F-Ph | 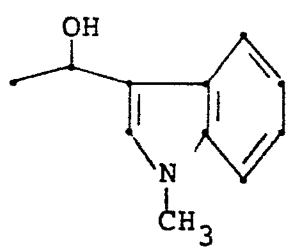 |
| H | 1 | o-F-Ph- | $NH(CH_2)_{1-3}$-3-indolyl |

TABLE 3 (cont'd)

| $X^1$ | r | $R^2$ | $R^3$ |
|---|---|---|---|
| H | 1 | o-F-Ph | $-NHCH_2-(CH_2)_2$-3-indolyl<br>COOEt |
| H | 1 | o-F-Ph | $-CH_2NH(CH_2)_2$-3-indolyl |
| H | 1 | o-F-Ph | $-NHCO-(CH_2)_{0-2}$-2-indolyl |
| H | 1 | o-F-Ph | $-NHCO(CH_2)_{0-2}$-3-indolyl |
| H | 1 | o-F-Ph | -CO-2-indolyl |
| H | 1 | o-F-Ph | -CO-3-indolyl |
| H | 1 | o-F-Ph | NHCO-p-halo-phenyl |
| H | 1 | o-F-Ph | NHCO-m-halo-phenyl |
| H | 1 | o-F-Ph | NHCO-2-benzofuranyl |
| H | 1 | o-F-Ph | 2-(3-methylindenyl) |
| H | 1 | o-F-Ph | phenylethenyl |
| H | 1 | o-F-Ph | NHCONH-p-halo-phenyl |
| H | 1 | p-Cl-Ph | $-CH_2$-2-indolyl |
| H | 1 | p-Cl-Ph | $-CH_2$-3-indolyl |

TABLE 3 (cont'd)

| $X^1$ | r | $R^2$ | $R^3$ |
|---|---|---|---|
| H | 1 | p-Cl-Ph | $-NH(CH_2)_{1-3}$-3-indolyl |
| H | 1 | p-Cl-Ph | -CHOH-1-methylindol-3-yl |
| H | 1 | p-Cl-Ph | $-NHCO-(CH_2)_{0-2}$-2-indolyl |
| H | 1 | p-Cl-Ph | NHCO-p-halo-phenyl |
| H | 1 | p-Cl-Ph | NHCO-m-halo-phenyl |
| H | 1 | p-Cl-Ph | NHCO-2-benzofuranyl |
| H | 1 | p-Cl-Ph | 2-(3-methylindenyl) |
| H | 1 | p-Cl-Ph | phenylethenyl |
| H | 1 | p-Cl-Ph | NHCONH-p-halo-phenyl |
| H | 1 | phenyl | $-CH_2$-2-indolyl |
| H | 1 | phenyl | $-CH_2$-3-indolyl |
| H | 1 | phenyl | $-NHCO(CH_2)_{0-2}$-indolyl |
| H | 1 | phenyl | -CHOH-1-methylindol-3-yl |
| H | 1 | phenyl | $-NH(CH_2)_{1-3}$-3-indolyl |

TABLE 3 (cont'd)

| $X^1$ | r | $R^2$ | $R^3$ |
|---|---|---|---|
| H | 1 | phenyl | NHCO-p-halo-phenyl |
| H | 1 | phenyl | NHCO-m-halo-phenyl |
| H | 1 | phenyl | NHCO-2-benzofuranyl |
| H | 1 | phenyl | 2-(3-methylindenyl) |
| H | 1 | phenyl | phenylethenyl |
| H | 1 | phenyl | NHCONH-p-halo-phenyl |
| H | 1 | p-F-Ph | $-CH_2$-2-indolyl |
| H | 1 | p-F-Ph | $-CH_2$-3-indolyl |
| H | 1 | p-F-Ph | $-NHCO(CH_2)_{0-2}$-2-indolyl |
| H | 1 | p-F-Ph | -CHOH-1-methylindol-3-yl |
| H | 1 | p-F-Ph | $-NH(CH_2)_{1-3}$-3-indolyl |
| H | 1 | p-F-Ph | NHCO-p-halo-phenyl |
| H | 1 | p-F-Ph | NHCO-m-halo-phenyl |
| H | 1 | p-F-Ph | NHCO-2-benzofuranyl |

TABLE 3 (cont'd)

| $X^1$ | r | $R^2$ | $R^3$ |
|---|---|---|---|
| H | 1 | p-F-Ph | 2-(3-methylindenyl) |
| H | 1 | p-F-Ph | phenylethenyl |
| H | 1 | p-F-Ph | NHCONH-p-halo-phenyl |
| H | 1 | 2,4-di-Cl-Ph | $-CH_2$-2-indolyl |
| H | 1 | 2,4-di-Cl-Ph | $-CH_2$-3-indolyl |
| H | 1 | 2,4-di-Cl-Ph | $-NHCO(CH_2)_{0-2}$-2-indolyl |
| H | 1 | 2,4-di-Cl-Ph | -CHOH-1-methylindol-3-yl |
| H | 1 | 2,4-di-Cl-Ph | $-NH(CH_2)_{1-3}$-3-indolyl |
| H | 1 | 2,4-di-Cl-Ph | NHCO-p-halo-phenyl |
| H | 1 | 2,4-di-Cl-Ph | NHCO-m-halo-phenyl |
| H | 1 | 2,4-di-Cl-Ph | NHCO-2-benzofuranyl |
| H | 1 | 2,4-di-Cl-Ph | 2-(3-methylindenyl) |
| H | 1 | 2,4-di-Cl-Ph | phenylethenyl |
| H | 1 | 2,4-di-Cl-Ph | NHCONH-p-halo-phenyl |

TABLE 3 (cont'd)

| $X^1$ | r | $R^2$ | $R^3$ |
|---|---|---|---|
| H | 1 | 2,6-di-F-Ph | $-CH_2$-2-indolyl |
| H | 1 | 2,6-di-F-Ph | $-CH_2$-3-indolyl |
| H | 1 | 2,6-di-F-Ph | $-NHCO(CH_2)_{0-2}$-2-indolyl |
| H | 1 | 2,6-di-F-Ph | -CHOH-1-methylindol-3-yl |
| H | 1 | 2,6-di-F-Ph | $-NH(CH_2)_{1-3}$-3-indolyl |
| H | 1 | 2,6-di-F-Ph | NHCO-p-halo-phenyl |
| H | 1 | 2,6-di-F-Ph | NHCO-m-halo-phenyl |
| H | 1 | 2,6-di-F-Ph | NHCO-2-benzofuranyl |
| H | 1 | 2,6-di-F-Ph | 2-(3-methylindenyl) |
| H | 1 | 2,6-di-F-Ph | phenylethenyl |
| H | 1 | 2,6-di-F-Ph | NHCONH-p-halo-phenyl |
| H | 1 | $CH_2COO$-t-butyl | $-CH_2$-2-indolyl |
| H | 1 | $CH_2COO$-t-butyl | $-CH_2$-3-indolyl |
| H | 1 | $CH_2COO$-t-butyl | $-NHCO(CH_2)_{0-2}$-indolyl |

TABLE 3 (cont'd)

| $X^1$ | r | $R^2$ | $R^3$ |
|---|---|---|---|
| H | 1 | $CH_2COO$-t-butyl | -CHOH-1-methylindol-3-yl |
| H | 1 | $CH_2COO$-t-butyl | $-NH(CH_2)_{1-3}$-3-indolyl |
| H | 1 | $CH_2COO$-t-butyl | NHCO-p-halo-phenyl |
| H | 1 | $CH_2COO$-t-butyl | NHCO-m-halo-phenyl |
| H | 1 | $CH_2COO$-t-butyl | NHCO-2-benzofuranyl |
| H | 1 | $CH_2COO$-t-butyl | 2-(3-methylindenyl) |
| H | 1 | $CH_2COO$-t-butyl | phenylethenyl |
| H | 1 | $CH_2COO$-t-butyl | NHCONH-p-halo-phenyl |
| H | 1 | $-CH_2COOEt$ | $-CH_2$-2-indolyl |
| H | 1 | $-CH_2COOEt$ | $-CH_2$-3-indolyl |
| H | 1 | $-CH_2COOEt$ | $-NHCO(CH_2)_{0-2}$-indolyl |
| H | 1 | $-CH_2COOEt$ | -CHOH-1-methylindol-3-yl |
| H | 1 | $-CH_2COOEt$ | $-NH(CH_2)_{1-3}$-3-indolyl |
| H | 1 | $-CH_2COOEt$ | NHCO-p-halo-phenyl |

TABLE 3 (cont'd)

| $X^1$ | r | $R^2$ | $R^3$ |
|---|---|---|---|
| H | 1 | $-CH_2COOEt$ | NHCO-m-halo-phenyl |
| H | 1 | $-CH_2COOEt$ | NHCO-2-benzofuranyl |
| H | 1 | $-CH_2COOEt$ | 2-(3-methylindenyl) |
| H | 1 | $-CH_2COOEt$ | phenylethenyl |
| H | 1 | $-CH_2COOEt$ | NHCONH-p-halo-phenyl |
| Cl | 1 | p-Cl-Ph | $-CH_2$-2-indolyl |
| Cl | 1 | p-Cl-Ph | $-CH_2$-3-indolyl |
| Cl | 1 | p-Cl-Ph | $-NHCO(CH_2)_{0-2}$-2-indolyl |
| Cl | 1 | p-Cl-Ph | -CHOH-1-methylidol-3-yl |
| Cl | 1 | p-Cl-Ph | $-NH(CH_2)_{1-3}$-3-indolyl |
| Cl | 1 | p-Cl-Ph | NHCO-p-halo-phenyl |
| Cl | 1 | p-Cl-Ph | NHCO-m-halo-phenyl |
| Cl | 1 | p-Cl-Ph | NHCO-2-benzofuranyl |
| Cl | 1 | p-Cl-Ph | 2-(3-methylindenyl) |

TABLE 3 (cont'd)

| $X^1$ | r | $R^2$ | $R^3$ |
|---|---|---|---|
| Cl | 1 | p-Cl-Ph | phenylethenyl |
| Cl | 1 | p-Cl-Ph | NHCONH-p-halo-phenyl |
| Cl | 1 | Ph | $-CH_2$-2-indolyl |
| Cl | 1 | Ph | $-CH_2$-3-indolyl |
| Cl | 1 | Ph | $-NHCO(CH_2)_{0-2}$-2-indolyl |
| Cl | 1 | Ph | -CHOH-1-methylindol-3-yl |
| Cl | 1 | Ph | $-NH(CH_2)_{1-3}$-3-indolyl |
| Cl | 1 | Ph | NHCO-p-halo-phenyl |
| Cl | 1 | Ph | NHCO-m-halo-phenyl |
| Cl | 1 | Ph | NHCO-2-benzofuranyl |
| Cl | 1 | Ph | 2-(3-methylindenyl) |
| Cl | 1 | Ph | phenylethenyl |
| Cl | 1 | Ph | NHCONH-p-halo-phenyl |
| Cl | 1 | o-F-Ph | $-CH_2$-2-indolyl |

TABLE 3 (cont'd)

| $X^1$ | r | $R^2$ | $R^3$ |
|---|---|---|---|
| Cl | 1 | o-F-Ph | $-CH_2$-3-indolyl |
| Cl | 1 | o-F-Ph | $-NHCO(CH_2)_{0-2}$-2-indolyl |
| Cl | 1 | o-F-Ph | -CHOH-1-methylindol-3-yl |
| Cl | 1 | o-F-Ph | $-NH(CH_2)_{1-3}$-3-indolyl |
| Cl | 1 | o-F-Ph | NHCO-p-halo-phenyl |
| Cl | 1 | o-F-Ph | NHCO-m-halo-phenyl |
| Cl | 1 | o-F-Ph | NHCO-2-benzofuranyl |
| Cl | 1 | o-F-Ph | 2-(3-methylindenyl) |
| Cl | 1 | o-F-Ph | phenylethenyl |
| Cl | 1 | o-F-Ph | NHCONH-p-halo-phenyl |
| Cl | 1 | p-F-Ph | $-CH_2$-2-indolyl |
| Cl | 1 | p-F-Ph | $-CH_2$-3-indolyl |
| Cl | 1 | p-F-Ph | $-NHCO(CH_2)_{0-2}$-2-indolyl |
| Cl | 1 | p-F-Ph | -CHOH-1-methylindol-3-yl |

TABLE 3 (cont'd)

| $X^1$ | r | $R^2$ | $R^3$ |
|---|---|---|---|
| Cl | 1 | p-F-Ph | $-NH(CH_2)_{1-3}$-3-indolyl |
| Cl | 1 | p-F-Ph | NHCO-p-halo-phenyl |
| Cl | 1 | p-F-Ph | NHCO-m-halo-phenyl |
| Cl | 1 | p-F-Ph | NHCO-2-benzofuranyl |
| Cl | 1 | p-F-Ph | 2-(3-methylindenyl) |
| Cl | 1 | p-F-Ph | phenylethenyl |
| Cl | 1 | p-F-Ph | NHCONH-p-halo-phenyl |
| F | 1 | 2,4-di-Cl-Ph | $-CH_2$-2-indolyl |
| F | 1 | 2,4-di-Cl-Ph | $-CH_2$-3-indolyl |
| F | 1 | 2,4-di-Cl-Ph | $-NHCO(CH_2)_{0-2}$-2-indolyl |
| F | 1 | 2,4-di-Cl-Ph | -CHOH-1-methylindol-3-yl |
| F | 1 | 2,4-di-Cl-Ph | $-NH(CH_2)_{1-3}$-3-indolyl |
| F | 1 | 2,4-di-Cl-Ph | NHCO-p-halo-phenyl |
| F | 1 | 2,4-di-Cl-Ph | NHCO-m-halo-phenyl |

TABLE 3 (cont'd)

| $X^1$ | r | $R^2$ | $R^3$ |
|---|---|---|---|
| F | 1 | 2,4-di-Cl-Ph | NHCO-2-benzofuranyl |
| F | 1 | 2,4-di-Cl-Ph | 2-(3-methylindenyl) |
| F | 1 | 2,4-di-Cl-Ph | phenylethenyl |
| F | 1 | 2,4-di-Cl-Ph | NHCONH-p-halo-phenyl |
| F | 1 | 2,6-di-F-Ph | $-CH_2$-2-indolyl |
| F | 1 | 2,6-di-F-Ph | $-CH_2$-3-indolyl |
| F | 1 | 2,6-di-F-Ph | $-NHCO(CH_2)_{0-2}$-2-indolyl |
| F | 1 | 2,6-di-F-Ph | -CHOH-1-methylindol-3-yl |
| F | 1 | 2,6-di-F-Ph | $-NH(CH_2)_{1-3}$-3-indolyl |
| F | 1 | 2,6-di-F-Ph | NHCO-p-halo-phenyl |
| F | 1 | 2,6-di-F-Ph | NHCO-m-halo-phenyl |
| F | 1 | 2,6-di-F-Ph | NHCO-2-benzofuranyl |
| F | 1 | 2,6-di-F-Ph | 2-(3-methylindenyl) |
| F | 1 | 2,6-di-F-Ph | phenylethenyl |

TABLE 3 (cont'd)

| $X^1$ | r | $R^2$ | $R^3$ |
|---|---|---|---|
| F | 1 | 2,6-di-F-Ph | NHCONH-p-halo-phenyl |
| F | 1 | COO-t-butyl | $-CH_2$-2-indolyl |
| F | 1 | COO-t-butyl | $-CH_2$-3-indolyl |
| F | 1 | COO-t-butyl | $-NHCO(CH_2)_{0-2}$-2-indolyl |
| F | 1 | COO-t-butyl | -CHOH-1-methylindol-3-yl |
| F | 1 | COO-t-butyl | $-NH(CH_2)_{1-3}$-3-indolyl |
| F | 1 | COO-t-butyl | NHCO-p-halo-phenyl |
| F | 1 | COO-t-butyl | NHCO-m-halo-phenyl |
| F | 1 | COO-t-butyl | NHCO-2-benzofuranyl |
| F | 1 | COO-t-butyl | 2-(3-methylindenyl) |
| F | 1 | COO-t-butyl | phenylethenyl |
| F | 1 | COO-t-butyl | NHCONH-p-halo-phenyl |
| F | 1 | $-CH_2COOEt$ | $-CH_2$-2-indolyl |
| F | 1 | $-CH_2COOEt$ | $-CH_2$-3-indolyl |

TABLE 3 (cont'd)

| $X^1$ | r | $R^2$ | $R^3$ |
|---|---|---|---|
| F | 1 | $-CH_2COOEt$ | $-NHCO(CH_2)_{0-2}$-2-indolyl |
| F | 1 | $-CH_2COOEt$ | -CHOH-1-methylindol-3-yl |
| F | 1 | $-CH_2COOEt$ | $-NH(CH_2)_{1-3}$-3-indolyl |
| F | 1 | $-CH_2COOEt$ | NHCO-p-halo-phenyl |
| F | 1 | $-CH_2COOEt$ | NHCO-m-halo-phenyl |
| F | 1 | $-CH_2COOEt$ | NHCO-2-benzofuranyl |
| F | 1 | $-CH_2COOEt$ | 2-(3-methylindenyl) |
| F | 1 | $-CH_2COOEt$ | phenylethenyl |
| F | 1 | $-CH_2COOEt$ | NHCONH-p-halo-phenyl |
| $CF_3$ | 1 | p-Cl-Ph | $-CH_2$-2-indolyl |
| $CF_3$ | 1 | p-Cl-Ph | $-CH_2$-3-indolyl |
| $CF_3$ | 1 | p-Cl-Ph | $-NHCO(CH_2)_{0-2}$-2-indolyl |
| $CF_3$ | 1 | p-Cl-Ph | -CHOH-1-methylindol-3-yl |
| $CF_3$ | 1 | p-Cl-Ph | $-NH(CH_2)_{1-3}$-3-indolyl |

TABLE 3 (cont'd)

| $X^1$ | r | $R^2$ | $R^3$ |
|---|---|---|---|
| $CF_3$ | 1 | p-Cl-Ph | NHCO-p-halo-phenyl |
| $CF_3$ | 1 | p-Cl-Ph | NHCO-m-halo-phenyl |
| $CF_3$ | 1 | p-Cl-Ph | NHCO-2-benzofuranyl |
| $CF_3$ | 1 | p-Cl-Ph | 2-(3-methylindenyl) |
| $CF_3$ | 1 | p-Cl-Ph | phenylethenyl |
| $CF_3$ | 1 | p-Cl-Ph | NHCONH-p-halo-phenyl |
| $CF_3$ | 1 | Ph | $-CH_2$-2-indolyl |
| $CF_3$ | 1 | Ph | $-CH_2$-3-indolyl |
| $CF_3$ | 1 | Ph | $-NHCO(CH_2)_{0-2}$-2-indolyl |
| $CF_3$ | 1 | Ph | -CHOH-1-methylindol-3-yl |
| $CF_3$ | 1 | Ph | $-NH(CH_2)_{1-3}$-3-indolyl |
| $CF_3$ | 1 | Ph | NHCO-p-halo-phenyl |
| $CF_3$ | 1 | Ph | NHCO-m-halo-phenyl |
| $CF_3$ | 1 | Ph | NHCO-2-benzofuranyl |

TABLE 3 (cont'd)

| $X^1$ | r | $R^2$ | $R^3$ |
|---|---|---|---|
| F | 1 | $-CH_2COOEt$ | $-NHCO(CH_2)_{0-2}$-2-indolyl |
| F | 1 | $-CH_2COOEt$ | -CHOH-1-methylindol-3-yl |
| F | 1 | $-CH_2COOEt$ | $-NH(CH_2)_{1-3}$-3-indolyl |
| F | 1 | $-CH_2COOEt$ | NHCO-p-halo-phenyl |
| F | 1 | $-CH_2COOEt$ | NHCO-m-halo-phenyl |
| F | 1 | $-CH_2COOEt$ | NHCO-2-benzofuranyl |
| F | 1 | $-CH_2COOEt$ | 2-(3-methylindenyl) |
| F | 1 | $-CH_2COOEt$ | phenylethenyl |
| F | 1 | $-CH_2COOEt$ | NHCONH-p-halo-phenyl |
| $CF_3$ | 1 | p-Cl-Ph | $-CH_2$-2-indolyl |
| $CF_3$ | 1 | p-Cl-Ph | $-CH_2$-3-indolyl |
| $CF_3$ | 1 | p-Cl-Ph | $-NHCO(CH_2)_{0-2}$-2-indolyl |
| $CF_3$ | 1 | p-Cl-Ph | -CHOH-1-methylindol-3-yl |
| $CF_3$ | 1 | p-Cl-Ph | $-NH(CH_2)_{1-3}$-3-indolyl |

TABLE 3 (cont'd)

| $X^1$ | r | $R^2$ | $R^3$ |
|---|---|---|---|
| $CF_3$ | 1 | p-Cl-Ph | NHCO-p-halo-phenyl |
| $CF_3$ | 1 | p-Cl-Ph | NHCO-m-halo-phenyl |
| $CF_3$ | 1 | p-Cl-Ph | NHCO-2-benzofuranyl |
| $CF_3$ | 1 | p-Cl-Ph | 2-(3-methylindenyl) |
| $CF_3$ | 1 | p-Cl-Ph | phenylethenyl |
| $CF_3$ | 1 | p-Cl-Ph | NHCONH-p-halo-phenyl |
| $CF_3$ | 1 | Ph | $-CH_2$-2-indolyl |
| $CF_3$ | 1 | Ph | $-CH_2$-3-indolyl |
| $CF_3$ | 1 | Ph | $-NHCO(CH_2)_{0-2}$-2-indolyl |
| $CF_3$ | 1 | Ph | -CHOH-1-methylindol-3-yl |
| $CF_3$ | 1 | Ph | $-NH(CH_2)_{1-3}$-3-indolyl |
| $CF_3$ | 1 | Ph | NHCO-p-halo-phenyl |
| $CF_3$ | 1 | Ph | NHCO-m-halo-phenyl |
| $CF_3$ | 1 | Ph | NHCO-2-benzofuranyl |

TABLE 3 (cont'd)

| $X^1$ | r | $R^2$ | $R^3$ |
|---|---|---|---|
| $CF_3$ | 1 | Ph | 2-(3-methylindenyl) |
| $CF_3$ | 1 | Ph | phenylethenyl |
| $CF_3$ | 1 | Ph | NHCONH-p-halo-phenyl |
| $CF_3$ | 1 | o-F-Ph | $-CH_2$-2-indolyl |
| $CF_3$ | 1 | o-F-Ph | $-CH_2$-3-indolyl |
| $CF_3$ | 1 | o-F-Ph | $-NHCO(CH_2)_{0-2}$-2-indolyl |
| $CF_3$ | 1 | o-F-Ph | -CHOH-1-methylindol-3-yl |
| $CF_3$ | 1 | o-F-Ph | $-NH(CH_2)_{1-3}$-3-indolyl |
| $CF_3$ | 1 | o-F-Ph | NHCO-p-halo-phenyl |
| $CF_3$ | 1 | o-F-Ph | NHCO-m-halo-phenyl |
| $CF_3$ | 1 | o-F-Ph | NHCO-2-benzofuranyl |
| $CF_3$ | 1 | o-F-Ph | 2-(3-methylindenyl) |
| $CF_3$ | 1 | o-F-Ph | phenylethenyl |
| $CF_3$ | 1 | o-F-Ph | NHCONH-p-halo-phenyl |

TABLE 3 (cont'd)

| $X^1$ | r | $R^2$ | $R^3$ |
|---|---|---|---|
| $CF_3$ | 1 | p-F-Ph | $-CH_2$-2-indolyl |
| $CF_3$ | 1 | p-F-Ph | $-CH_2$-3-indolyl |
| $CF_3$ | 1 | p-F-Ph | $-NHCO(CH_2)_{0-2}$-2-indolyl |
| $CF_3$ | 1 | p-F-Ph | -CHOH-1-methylindol-3-yl |
| $CF_3$ | 1 | p-F-Ph | $-NH(CH_2)_{1-3}$-3-indolyl |
| $CF_3$ | 1 | p-F-Ph | NHCO-p-halo-phenyl |
| $CF_3$ | 1 | p-F-Ph | NHCO-m-halo-phenyl |
| $CF_3$ | 1 | p-F-Ph | NHCO-2-benzofuranyl |
| $CF_3$ | 1 | p-F-Ph | 2-(3-methylindenyl) |
| $CF_3$ | 1 | p-F-Ph | phenylethenyl |
| $CF_3$ | 1 | p-F-Ph | NHCONH-p-halo-phenyl |
| $NO_2$ | 1 | p-Cl-Ph | $-CH_2$-2-indolyl |
| $NO_2$ | 1 | p-Cl-Ph | $-CH_2$-3-indolyl |
| $NO_2$ | 1 | p-Cl-Ph | $-NHCO(CH_2)_{0-2}$-2-indolyl |

TABLE 3 (cont'd)

| $X^1$ | r | $R^2$ | $R^3$ |
|---|---|---|---|
| $NO_2$ | 1 | p-Cl-Ph | -CHOH-1-methylindol-3-yl |
| $NO_2$ | 1 | p-Cl-Ph | $-NH(CH_2)_{1-3}$-3-indolyl |
| $NO_2$ | 1 | p-Cl-Ph | NHCO-p-halo-phenyl |
| $NO_2$ | 1 | p-Cl-Ph | NHCO-m-halo-phenyl |
| $NO_2$ | 1 | p-Cl-Ph | NHCO-2-benzofuranyl |
| $NO_2$ | 1 | p-Cl-Ph | 2-(3-methylindenyl) |
| $NO_2$ | 1 | p-Cl-Ph | phenylethenyl |
| $NO_2$ | 1 | p-Cl-Ph | NHCONH-p-halo-phenyl |
| $NO_2$ | 1 | Ph | $-CH_2$-2-indolyl |
| $NO_2$ | 1 | Ph | $-CH_2$-3-indolyl |
| $NO_2$ | 1 | Ph | $-NHCO(CH_2)_{0-2}$-2-indolyl |
| $NO_2$ | 1 | Ph | -CHOH-1-methylindol-3-yl |
| $NO_2$ | 1 | Ph | $-NH(CH_2)_{1-3}$-3-indolyl |
| $NO_2$ | 1 | Ph | NHCO-p-halo-phenyl |

TABLE 3 (cont'd)

| $X^1$ | r | $R^2$ | $R^3$ |
|---|---|---|---|
| $NO_2$ | 1 | Ph | NHCO-m-halo-phenyl |
| $NO_2$ | 1 | Ph | NHCO-2-benzofuranyl |
| $NO_2$ | 1 | Ph | 2-(3-methylindenyl) |
| $NO_2$ | 1 | Ph | phenylethenyl |
| $NO_2$ | 1 | Ph | NHCONH-p-halo-phenyl |
| $NO_2$ | 1 | o-F-Ph | $-CH_2$-2-indolyl |
| $NO_2$ | 1 | o-F-Ph | $-CH_2$-3-indolyl |
| $NO_2$ | 1 | o-F-Ph | $-NHCO(CH_2)_{0-2}$-2-indolyl |
| $NO_2$ | 1 | o-F-Ph | -CHOH-1-methylindol-3-yl |
| $NO_2$ | 1 | o-F-Ph | $-NH(CH_2)_{1-3}$-3-indolyl |
| $NO_2$ | 1 | o-F-Ph | NHCO-p-halo-phenyl |
| $NO_2$ | 1 | o-F-Ph | NHCO-m-halo-phenyl |
| $NO_2$ | 1 | o-F-Ph | NHCO-2-benzofuranyl |
| $NO_2$ | 1 | o-F-Ph | 2-(3-methylindenyl) |

TABLE 3 (cont'd)

| $X^1$ | r | $R^2$ | $R^3$ |
|---|---|---|---|
| $NO_2$ | 1 | o-F-Ph | phenylethenyl |
| $NO_2$ | 1 | o-F-Ph | NHCONH-p-halo-phenyl |
| $NO_2$ | 1 | p-F-Ph | $-CH_2$-2-indolyl |
| $NO_2$ | 1 | p-F-Ph | $-CH_2$-3-indolyl |
| $NO_2$ | 1 | p-F-Ph | $-NHCO(CH_2)_{0-2}$-2-indolyl |
| $NO_2$ | 1 | p-F-Ph | -CHOH-1-methylindol-3-yl |
| $NO_2$ | 1 | p-F-Ph | $-NH(CH_2)_{1-3}$-3-indolyl |
| $NO_2$ | 1 | p-F-Ph | NHCO-p-halo-phenyl |
| $NO_2$ | 1 | p-F-Ph | NHCO-m-halo-phenyl |
| $NO_2$ | 1 | p-F-Ph | NHCO-2-benzofuranyl |
| $NO_2$ | 1 | p-F-Ph | 2-(3-methylindenyl) |
| $NO_2$ | 1 | p-F-Ph | phenylethenyl |
| $NO_2$ | 1 | p-F-Ph | NHCONH-p-halo-phenyl |
| $CH_3$ | 1 | phenyl | $-CH_2$-2-indolyl |

TABLE 3 (cont'd)

| $X^1$ | r | $R^2$ | $R^3$ |
|---|---|---|---|
| $CH_3$ | 1 | phenyl | $-CH_2$-3-indolyl |
| $CH_3$ | 1 | phenyl | $-NHCO(CH_2)_{0-2}$-2-indolyl |
| $CH_3$ | 1 | phenyl | -CHOH-1-methylindol-3-yl |
| $CH_3$ | 1 | phenyl | $-NH(CH_2)_{1-3}$-3-indolyl |
| $CH_3$ | 1 | phenyl | NHCO-p-halo-phenyl |
| $CH_3$ | 1 | phenyl | NHCO-m-halo-phenyl |
| $CH_3$ | 1 | phenyl | NHCO-2-benzofuranyl |
| $CH_3$ | 1 | phenyl | 2-(3-methylindenyl) |
| $CH_3$ | 1 | phenyl | phenylethenyl |
| $CH_3$ | 1 | phenyl | NHCONH-p-halo-phenyl |
| H | 1 | o-F-Ph | $-CH_2$-2-indolyl |
| H | 1 | o-F-Ph | $-CH_2$-3-indolyl |
| H | 1 | o-F-Ph | $-NHCO(CH_2)_{0-2}$-2-indolyl |
| H | 1 | o-F-Ph | -CHOH-1-methylindol-3-yl |

TABLE 3 (cont'd)

| $X^1$ | r | $R^2$ | $R^3$ |
|---|---|---|---|
| H | 1 | o-F-Ph | $-NH(CH_2)_{1-3}$-3-indolyl |
| H | 1 | o-F-Ph | NHCO-p-halo-phenyl |
| H | 1 | o-F-Ph | NHCO-m-halo-phenyl |
| H | 1 | o-F-Ph | NHCO-2-benzofuranyl |
| H | 1 | o-F-Ph | 2-(3-methylindenyl) |
| H | 1 | o-F-Ph | phenylethenyl |
| H | 1 | o-F-Ph | NHCONH-p-halo-phenyl |
| H | 1 | p-Cl-Ph | $-CH_2$-2-indolyl |
| H | 1 | p-Cl-Ph | $-CH_2$-3-indolyl |
| H | 1 | p-Cl-Ph | $-NHCO(CH_2)_{0-2}$-2-indolyl |
| H | 1 | p-Cl-Ph | -CHOH-1-methylindol-3-yl |
| H | 1 | p-Cl-Ph | $-NH(CH_2)_{1-3}$-3-indolyl |
| H | 1 | p-Cl-Ph | NHCO-p-halo-phenyl |
| H | 1 | p-Cl-Ph | NHCO-m-halo-phenyl |

TABLE 3 (cont'd)

| $X^1$ | r | $R^2$ | $R^3$ |
|---|---|---|---|
| H | 1 | p-Cl-Ph | NHCO-2-benzofuranyl |
| H | 1 | p-Cl-Ph | 2-(3-methylindenyl) |
| H | 1 | p-Cl-Ph | phenylethenyl |
| H | 1 | p-Cl-Ph | NHCONH-p-halo-phenyl |
| H | 1 | Ph | -CO-3-thiophene |
| H | 1 | o-F-Ph | -CO-3-thiophene |
| H | 1 | p-Cl-Ph | -CO-3-thiophene |
| H | 1 | phenyl | -CO-3-thiophene |
| H | 1 | p-F-Ph | -CO-3-thiophene |
| H | 1 | 2,4-di-Cl-Ph | -CO-3-thiophene |
| H | 1 | 2,6-di-F-Ph | -CO-3-thiophene |
| H | 1 | $-CH_2COO$-t-butyl | -CO-3-thiophene |
| H | 1 | $-CH_2COOEt$ | -CO-3-thiophene |

TABLE 3 (cont'd)

| $X^1$ | r | $R^2$ | $R^3$ |
|---|---|---|---|
| H | 1 | o-F-Ph | $CH_2$-2 or 3-(1-methyl indolyl) |
| H | 1 | $-CH_2COO$-t-butyl | $CH_2$-2 or 3-(1-methyl indolyl) |
| H | 1 | $-CH_2COOEt$ | $CH_2$-2 or 3-(1-methyl indolyl) |
| H | 1 | Ph | $CH_2$-2 or 3-(1-methyl indolyl) |
| H | 1 | o-F-Ph | $NHCO(CH_2)_{0-2}$-2-indolyl |
| H | 1 | $-CH_2COO$-t-butyl | $NHCO(CH_2)_{0-2}$-2-indolyl |
| H | 1 | $-CH_2COOEt$ | $NHCO(CH_2)_{0-2}$-2-indolyl |
| H | 1 | Ph | $NHCO(CH_2)_{0-2}$-2-indolyl |
| H | 1 | o-F-Ph | $NHCO(CH_2)_{0-2}$-2 or 3-(1-methylindolyl) |
| H | 1 | $-CH_2COO$-t-butyl | $NHCO(CH_2)_{0-2}$-2 or 3-(1-methylindolyl) |
| H | 1 | $-CH_2COOEt$ | $NHCO(CH_2)_{0-2}$-2 or 3-(1-methylindolyl) |

TABLE 3 (cont'd)

| $X^1$ | r | $R^2$ | $R^3$ |
|---|---|---|---|
| H | 1 | Ph | $NHCO(CH_2)_{0-2}$-2 or 3-(1-methylindolyl) |
| H | 1 | o-F-Ph | Boc-Trp-NH |
| H | 1 | $-CH_2COO$-t-butyl | Boc-Trp-NH |
| H | 1 | $-CH_2COOEt$ | Boc-Trp-NH |
| H | 1 | Ph | Boc-Trp-NH |
| H | 1 | o-F-Ph | Cbz-Trp-NH |
| H | 1 | $-CH_2COO$-t-butyl | Cbz-Trp-NH |
| H | 1 | $-CH_2COOEt$ | Cbz-Trp-NH |
| H | 1 | Ph | Cbz-Trp-NH |
| H | 1 | o-F-Ph | $NH(CH_2)_{1-3}$-2-indolyl |
| H | 1 | $-CH_2COO$-t-butyl | $NH(CH_2)_{1-3}$-2-indolyl |
| H | 1 | $-CH_2COOEt$ | $NH(CH_2)_{1-3}$-2-indolyl |
| H | 1 | Ph | $NH(CH_2)_{1-3}$-2-indolyl |

TABLE 3 (cont'd)

| $X^1$ | r | $R^2$ | $R^3$ |
|---|---|---|---|
| H | 1 | o-F-Ph | $NH(CH_2)_{1-3}$-2 or 3-(1-methylindolyl) |
| H | 1 | $-CH_2COO$-t-butyl | $NH(CH_2)_{1-3}$-2 or 3-(1-methylindolyl) |
| H | 1 | $-CH_2COOEt$ | $NH(CH_2)_{1-3}$-2 or 3-(1-methylindolyl) |
| H | 1 | Ph | $NH(CH_2)_{1-3}$-2 or 3-(1-methylindolyl) |
| H | 1 | o-F-Ph | CHOH-2 or 3-indolyl |
| H | 1 | $-CH_2COO$-t-butyl | CHOH-2 or 3-indolyl |
| H | 1 | $-CH_2COOEt$ | CHOH-2 or 3-indolyl |
| H | 1 | Ph | CHOH-2 or 3-indolyl |
| H | 1 | o-F-Ph | CHOH-2-(1-methylindolyl) |
| H | 1 | $-CH_2COO$-t-butyl | CHOH-2-(1-methylindolyl) |
| H | 1 | $-CH_2COOEt$ | CHOH-2-(1-methylindolyl) |
| H | 1 | Ph | CHOH-2-(1-methylindolyl) |

TABLE 3 (cont'd)

| $X^1$ | r | $R^2$ | $R^3$ |
|---|---|---|---|
| H | 1 | $-CH_2COO$-t-butyl | CO-2-thiophene |
| H | 1 | $-CH_2COOEt$ | CO-2-thiophene |
| H | 1 | Ph | CO-2-thiophene |
| H | 1 | o-F-Ph | $NHCH(COOEt)(CH_2)_2$-2-indolyl |
| H | 1 | $-CH_2COO$-t-butyl | $NHCH(COOEt)(CH_2)_2$-2-indolyl |
| H | 1 | $-CH_2COOEt$ | $NHCH(COOEt)(CH_2)_2$-2-indolyl |
| H | 1 | Ph | $NHCH(COOEt)(CH_2)_2$-2-indolyl |
| H | 1 | o-F-Ph | $NHCH(COOEt)(CH_2)_2$-2 or 3-(1-methyl-indolyl) |
| H | 1 | $-CH_2COO$-t-butyl | $NHCH(COOEt)(CH_2)_2$-2 or 3-(1-methyl-indolyl) |
| H | 1 | $-CH_2COOEt$ | $NHCH(COOEt)(CH_2)_2$-2 or 3-(1-methyl-indolyl) |

TABLE 3 (cont'd)

| $X^1$ | r | $R^2$ | $R^3$ |
|---|---|---|---|
| H | 1 | Ph | COOEt<br>NHCH$(CH_2)_2$-2 or 3-(1-methyl-indolyl) |
| H | 1 | o-F-Ph | NHCH$(CH_2)_2$-2 or 3-indolyl<br>COO-t-Butyl |
| H | 1 | $-CH_2$COO-t-butyl | NHCH$(CH_2)_2$-2 or 3-indolyl<br>COO-t-Butyl |
| H | 1 | $-CH_2COOEt$ | NHCH$(CH_2)_2$-2 or 3-indolyl<br>COO-t-Butyl |
| H | 1 | Ph | NHCH$(CH_2)_2$-2 or 3-indolyl<br>COO-t-Butyl |
| H | 1 | o-F-Ph | COO-t-Butyl<br>NHCH$(CH_2)_2$-2 or 3-(1-methyl-indolyl) |
| H | 1 | $-CH_2$COO-t-butyl | COO-t-Butyl<br>NHCH$(CH_2)_2$-2 or 3-(1-methyl-indolyl) |
| H | 1 | $-CH_2COOEt$ | COO-t-Butyl<br>NHCH$(CH_2)_2$-2 or 3-(1-methyl-indolyl) |

TABLE 3 (cont'd)

| $X^1$ | r | $R^2$ | $R^3$ |
| --- | --- | --- | --- |
| H | 1 | Ph | NHCH(COO-t-Butyl)$(CH_2)_2$-2 or 3-(1-methyl-indolyl) |

TABLE 4

Compounds of the formula:

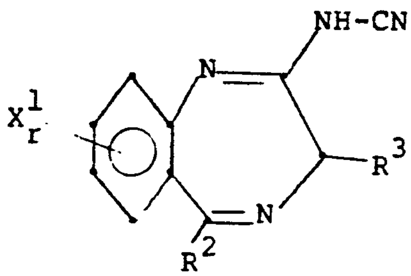

| $X^1$ | r | $R^2$ | $R^3$ |
|---|---|---|---|
| H | 1 | o-F-Ph | $-CH_2$-2-indolyl |
| H | 1 | o-F-Ph | $-CH_2$-3-indolyl |
| H | 1 | o-F-Ph | 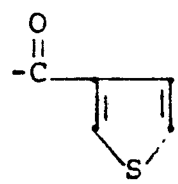 |
| H | 1 | o-F-Ph | 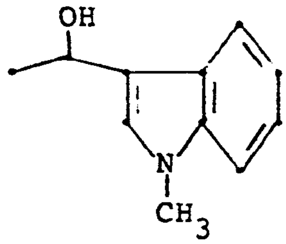 |
| H | 1 | o-F-Ph- | $NH(CH_2)_{1-3}$-3-indolyl |

TABLE 4 (cont'd)

| $X^1$ | r | $R^2$ | $R^3$ |
|---|---|---|---|
| H | 1 | o-F-Ph | $-NHCH_2-(CH_2)_2$-3-indolyl (COOEt on CH) |
| H | 1 | o-F-Ph | $-CH_2NH(CH_2)_2$-3-indolyl |
| H | 1 | o-F-Ph | $-NHCO-(CH_2)_{0-2}$-2-indolyl |
| H | 1 | o-F-Ph | $-NHCO(CH_2)_{0-2}$-3-indolyl |
| H | 1 | o-F-Ph | -CO-2-indolyl |
| H | 1 | o-F-Ph | -CO-3-indolyl |
| H | 1 | o-F-Ph | NHCO-p-halo-phenyl |
| H | 1 | o-F-Ph | NHCO-m-halo-phenyl |
| H | 1 | o-F-Ph | NHCO-2-benzofuranyl |
| H | 1 | o-F-Ph | 2-(3-methylindenyl) |
| H | 1 | o-F-Ph | phenylethenyl |
| H | 1 | o-F-Ph | NHCONH-p-halo-phenyl |
| H | 1 | p-Cl-Ph | $-CH_2$-2-indolyl |

TABLE 4 (cont'd)

| $X^1$ | r | $R^2$ | $R^3$ |
|---|---|---|---|
| H | 1 | p-Cl-Ph | $-CH_2$-3-indolyl |
| H | 1 | p-Cl-Ph | $-NH(CH_2)_{1-3}$-3-indolyl |
| H | 1 | p-Cl-Ph | -CHOH-1-methylindol-3-yl |
| H | 1 | p-Cl-Ph | $-NHCO-(CH_2)_{0-2}$-2-indolyl |
| H | 1 | p-Cl-Ph | NHCO-p-halo-phenyl |
| H | 1 | p-Cl-Ph | NHCO-m-halo-phenyl |
| H | 1 | p-Cl-Ph | NHCO-2-benzofuranyl |
| H | 1 | p-Cl-Ph | 2-(3-methylindenyl) |
| H | 1 | p-Cl-Ph | phenylethenyl |
| H | 1 | p-Cl-Ph | NHCONH-p-halo-phenyl |
| H | 1 | phenyl | $-CH_2$-2-indolyl |
| H | 1 | phenyl | $-CH_2$-3-indolyl |
| H | 1 | phenyl | $-NHCO(CH_2)_{0-2}$-3-indolyl |
| H | 1 | phenyl | -CHOH-1-methylindol-3-yl |

TABLE 4 (cont'd)

| $X^1$ | r | $R^2$ | $R^3$ |
|---|---|---|---|
| H | 1 | phenyl | $-NH(CH_2)_{1-3}$-3-indolyl |
| H | 1 | phenyl | NHCO-p-halo-phenyl |
| H | 1 | phenyl | NHCO-m-halo-phenyl |
| H | 1 | phenyl | NHCO-2-benzofuranyl |
| H | 1 | phenyl | 2-(3-methylindenyl) |
| H | 1 | phenyl | phenylethenyl |
| H | 1 | phenyl | NHCONH-p-halo-phenyl |
| H | 1 | p-F-Ph | $-CH_2$-2-indolyl |
| H | 1 | p-F-Ph | $-CH_2$-3-indolyl |
| H | 1 | p-F-Ph | $-NHCO(CH_2)_{0-2}$-2-indolyl |
| H | 1 | p-F-Ph | -CHOH-1-methylindol-3-yl |
| H | 1 | p-F-Ph | $-NH(CH_2)_{1-3}$-3-indolyl |
| H | 1 | p-F-Ph | NHCO-p-halo-phenyl |
| H | 1 | p-F-Ph | NHCO-m-halo-phenyl |

TABLE 4 (cont'd)

| $X^1$ | r | $R^2$ | $R^3$ |
|---|---|---|---|
| H | 1 | p-F-Ph | NHCO-2-benzofuranyl |
| H | 1 | p-F-Ph | 2-(3-methylindenyl) |
| H | 1 | p-F-Ph | phenylethenyl |
| H | 1 | p-F-Ph | NHCONH-p-halo-phenyl |
| H | 1 | 2,4-di-Cl-Ph | $-CH_2$-2-indolyl |
| H | 1 | 2,4-di-Cl-Ph | $-CH_2$-3-indolyl |
| H | 1 | 2,4-di-Cl-Ph | $-NHCO(CH_2)_{0-2}$-2-indolyl |
| H | 1 | 2,4-di-Cl-Ph | -CHOH-1-methylindol-3-yl |
| H | 1 | 2,4-di-Cl-Ph | $-NH(CH_2)_{1-3}$-3-indolyl |
| H | 1 | 2,4-di-Cl-Ph | NHCO-p-halo-phenyl |
| H | 1 | 2,4-di-Cl-Ph | NHCO-m-halo-phenyl |
| H | 1 | 2,4-di-Cl-Ph | NHCO-2-benzofuranyl |
| H | 1 | 2,4-di-Cl-Ph | 2-(3-methylindenyl) |
| H | 1 | 2,4-di-Cl-Ph | phenylethenyl |

TABLE 4 (cont'd)

| $X^1$ | r | $R^2$ | $R^3$ |
|---|---|---|---|
| H | 1 | 2,4-di-Cl-Ph | NHCONH-p-halo-phenyl |
| H | 1 | 2,6-di-F-Ph | $-CH_2$-2-indolyl |
| H | 1 | 2,6-di-F-Ph | $-CH_2$-3-indolyl |
| H | 1 | 2,6-di-F-Ph | $-NHCO(CH_2)_{0-2}$-2-indolyl |
| H | 1 | 2,6-di-F-Ph | -CHOH-1-methylindol-3-yl |
| H | 1 | 2,6-di-F-Ph | $-NH(CH_2)_{1-3}$-3-indolyl |
| H | 1 | 2,6-di-F-Ph | NHCO-p-halo-phenyl |
| H | 1 | 2,6-di-F-Ph | NHCO-m-halo-phenyl |
| H | 1 | 2,6-di-F-Ph | NHCO-2-benzofuranyl |
| H | 1 | 2,6-di-F-Ph | 2-(3-methylindenyl) |
| H | 1 | 2,6-di-F-Ph | phenylethenyl |
| H | 1 | 2,6-di-F-Ph | NHCONH-p-halo-phenyl |
| H | 1 | $CH_2COO$-t-butyl | $-CH_2$-2-indolyl |
| H | 1 | $CH_2COO$-t-butyl | $-CH_2$-3-indolyl |

TABLE 4 (cont'd)

| $X^1$ | r | $R^2$ | $R^3$ |
|---|---|---|---|
| H | 1 | $CH_2COO$-t-butyl | $-NHCO(CH_2)_{0-2}$-3-indolyl |
| H | 1 | $CH_2COO$-t-butyl | -CHOH-1-methylindol-3-yl |
| H | 1 | $CH_2COO$-t-butyl | $-NH(CH_2)_{1-3}$-3-indolyl |
| H | 1 | $CH_2COO$-t-butyl | NHCO-p-halo-phenyl |
| H | 1 | $CH_2COO$-t-butyl | NHCO-m-halo-phenyl |
| H | 1 | $CH_2COO$-t-butyl | NHCO-2-benzofuranyl |
| H | 1 | $CH_2COO$-t-butyl | 2-(3-methylindenyl) |
| H | 1 | $CH_2COO$-t-butyl | phenylethenyl |
| H | 1 | $CH_2COO$-t-butyl | NHCONH-p-halo-phenyl |
| H | 1 | $-CH_2COOEt$ | $-CH_2$-2-indolyl |
| H | 1 | $-CH_2COOEt$ | $-CH_2$-3-indolyl |
| H | 1 | $-CH_2COOEt$ | $-NHCO(CH_2)_{0-2}$-3-indolyl |
| H | 1 | $-CH_2COOEt$ | -CHOH-1-methylindol-3-yl |
| H | 1 | $-CH_2COOEt$ | $-NH(CH_2)_{1-3}$-3-indolyl |

TABLE 4 (cont'd)

| $X^1$ | r | $R^2$ | $R^3$ |
|---|---|---|---|
| H | 1 | $-CH_2COOEt$ | NHCO-p-halo-phenyl |
| H | 1 | $-CH_2COOEt$ | NHCO-m-halo-phenyl |
| H | 1 | $-CH_2COOEt$ | NHCO-2-benzofuranyl |
| H | 1 | $-CH_2COOEt$ | 2-(3-methylindenyl) |
| H | 1 | $-CH_2COOEt$ | phenylethenyl |
| H | 1 | $-CH_2COOEt$ | NHCONH-p-halo-phenyl |
| Cl | 1 | p-Cl-Ph | $-CH_2$-2-indolyl |
| Cl | 1 | p-Cl-Ph | $-CH_2$-3-indolyl |
| Cl | 1 | p-Cl-Ph | $-NHCO(CH_2)_{0-2}$-2-indolyl |
| Cl | 1 | p-Cl-Ph | -CHOH-1-methylidol-3-yl |
| Cl | 1 | p-Cl-Ph | $-NH(CH_2)_{1-3}$-3-indolyl |
| Cl | 1 | p-Cl-Ph | NHCO-p-halo-phenyl |
| Cl | 1 | p-Cl-Ph | NHCO-m-halo-phenyl |
| Cl | 1 | p-Cl-Ph | NHCO-2-benzofuranyl |

TABLE 4 (cont'd)

| $X^1$ | r | $R^2$ | $R^3$ |
|---|---|---|---|
| Cl | 1 | p-Cl-Ph | 2-(3-methylindenyl) |
| Cl | 1 | p-Cl-Ph | phenylethenyl |
| Cl | 1 | p-Cl-Ph | NHCONH-p-halo-phenyl |
| Cl | 1 | Ph | $-CH_2$-2-indolyl |
| Cl | 1 | Ph | $-CH_2$-3-indolyl |
| Cl | 1 | Ph | $-NHCO(CH_2)_{0-2}$-2-indolyl |
| Cl | 1 | Ph | -CHOH-1-methylindol-3-yl |
| Cl | 1 | Ph | $-NH(CH_2)_{1-3}$-3-indolyl |
| Cl | 1 | Ph | NHCO-p-halo-phenyl |
| Cl | 1 | Ph | NHCO-m-halo-phenyl |
| Cl | 1 | Ph | NHCO-2-benzofuranyl |
| Cl | 1 | Ph | 2-(3-methylindenyl) |
| Cl | 1 | Ph | phenylethenyl |
| Cl | 1 | Ph | NHCONH-p-halo-phenyl |

TABLE 4 (cont'd)

| $X^1$ | r | $R^2$ | $R^3$ |
|---|---|---|---|
| Cl | 1 | o-F-Ph | $-CH_2$-2-indolyl |
| Cl | 1 | o-F-Ph | $-CH_2$-3-indolyl |
| Cl | 1 | o-F-Ph | $-NHCO(CH_2)_{0-2}$-2-indolyl |
| Cl | 1 | o-F-Ph | -CHOH-1-methylindol-3-yl |
| Cl | 1 | o-F-Ph | $-NH(CH_2)_{1-3}$-3-indolyl |
| Cl | 1 | o-F-Ph | NHCO-p-halo-phenyl |
| Cl | 1 | o-F-Ph | NHCO-m-halo-phenyl |
| Cl | 1 | o-F-Ph | NHCO-2-benzofuranyl |
| Cl | 1 | o-F-Ph | 2-(3-methylindenyl) |
| Cl | 1 | o-F-Ph | phenylethenyl |
| Cl | 1 | o-F-Ph | NHCONH-p-halo-phenyl |
| Cl | 1 | p-F-Ph | $-CH_2$-2-indolyl |
| Cl | 1 | p-F-Ph | $-CH_2$-3-indolyl |
| Cl | 1 | p-F-Ph | $-NHCO(CH_2)_{0-2}$-2-indolyl |

TABLE 4 (cont'd)

| $X^1$ | r | $R^2$ | $R^3$ |
|---|---|---|---|
| Cl | 1 | p-F-Ph | -CHOH-1-methylindol-3-yl |
| Cl | 1 | p-F-Ph | $-NH(CH_2)_{1-3}$-3-indolyl |
| Cl | 1 | p-F-Ph | NHCO-p-halo-phenyl |
| Cl | 1 | p-F-Ph | NHCO-m-halo-phenyl |
| Cl | 1 | p-F-Ph | NHCO-2-benzofuranyl |
| Cl | 1 | p-F-Ph | 2-(3-methylindenyl) |
| Cl | 1 | p-F-Ph | phenylethenyl |
| Cl | 1 | p-F-Ph | NHCONH-p-halo-phenyl |
| F | 1 | 2,4-di-Cl-Ph | $-CH_2$-2-indolyl |
| F | 1 | 2,4-di-Cl-Ph | $-CH_2$-3-indolyl |
| F | 1 | 2,4-di-Cl-Ph | $-NHCO(CH_2)_{0-2}$-2-indolyl |
| F | 1 | 2,4-di-Cl-Ph | -CHOH-1-methylindol-3-yl |
| F | 1 | 2,4-di-Cl-Ph | $-NH(CH_2)_{1-3}$-3-indolyl |
| F | 1 | 2,4-di-Cl-Ph | NHCO-p-halo-phenyl |

TABLE 4 (cont'd)

| $X^1$ | r | $R^2$ | $R^3$ |
|---|---|---|---|
| F | 1 | 2,4-di-Cl-Ph | NHCO-m-halo-phenyl |
| F | 1 | 2,4-di-Cl-Ph | NHCO-2-benzofuranyl |
| F | 1 | 2,4-di-Cl-Ph | 2-(3-methylindenyl) |
| F | 1 | 2,4-di-Cl-Ph | phenylethenyl |
| F | 1 | 2,4-di-Cl-Ph | NHCONH-p-halo-phenyl |
| F | 1 | 2,6-di-F-Ph | $-CH_2$-2-indolyl |
| F | 1 | 2,6-di-F-Ph | $-CH_2$-3-indolyl |
| F | 1 | 2,6-di-F-Ph | $-NHCO(CH_2)_{0-2}$-2-indolyl |
| F | 1 | 2,6-di-F-Ph | -CHOH-1-methylindol-3-yl |
| F | 1 | 2,6-di-F-Ph | $-NH(CH_2)_{1-3}$-3-indolyl |
| F | 1 | 2,6-di-F-Ph | NHCO-p-halo-phenyl |
| F | 1 | 2,6-di-F-Ph | NHCO-m-halo-phenyl |
| F | 1 | 2,6-di-F-Ph | NHCO-2-benzofuranyl |
| F | 1 | 2,6-di-F-Ph | 2-(3-methylindenyl) |

TABLE 4 (cont'd)

| $X^1$ | r | $R^2$ | $R^3$ |
|---|---|---|---|
| F | 1 | 2,6-di-F-Ph | phenylethenyl |
| F | 1 | 2,6-di-F-Ph | NHCONH-p-halo-phenyl |
| F | 1 | COO-t-butyl | $-CH_2$-2-indolyl |
| F | 1 | COO-t-butyl | $-CH_2$-3-indolyl |
| F | 1 | COO-t-butyl | $-NHCO(CH_2)_{0-2}$-2-indolyl |
| F | 1 | COO-t-butyl | -CHOH-1-methylindol-3-yl |
| F | 1 | COO-t-butyl | $-NH(CH_2)_{1-3}$-3-indolyl |
| F | 1 | COO-t-butyl | NHCO-p-halo-phenyl |
| F | 1 | COO-t-butyl | NHCO-m-halo-phenyl |
| F | 1 | COO-t-butyl | NHCO-2-benzofuranyl |
| F | 1 | COO-t-butyl | 2-(3-methylindenyl) |
| F | 1 | COO-t-butyl | phenylethenyl |
| F | 1 | COO-t-butyl | NHCONH-p-halo-phenyl |
| F | 1 | $-CH_2COOEt$ | $-CH_2$-2-indolyl |

TABLE 4 (cont'd)

| $X^1$ | r | $R^2$ | $R^3$ |
|---|---|---|---|
| F | 1 | $-CH_2COOEt$ | $-CH_2$-3-indolyl |
| F | 1 | $-CH_2COOEt$ | $-NHCO(CH_2)_{0-2}$-2-indolyl |
| F | 1 | $-CH_2COOEt$ | -CHOH-1-methylindol-3-yl |
| F | 1 | $-CH_2COOEt$ | $-NH(CH_2)_{1-3}$-3-indolyl |
| F | 1 | $-CH_2COOEt$ | NHCO-p-halo-phenyl |
| F | 1 | $-CH_2COOEt$ | NHCO-m-halo-phenyl |
| F | 1 | $-CH_2COOEt$ | NHCO-2-benzofuranyl |
| F | 1 | $-CH_2COOEt$ | 2-(3-methylindenyl) |
| F | 1 | $-CH_2COOEt$ | phenylethenyl |
| F | 1 | $-CH_2COOEt$ | NHCONH-p-halo-phenyl |
| $CF_3$ | 1 | p-Cl-Ph | $-CH_2$-2-indolyl |
| $CF_3$ | 1 | p-Cl-Ph | $-CH_2$-3-indolyl |
| $CF_3$ | 1 | p-Cl-Ph | $-NHCO(CH_2)_{0-2}$-2-indolyl |
| $CF_3$ | 1 | p-Cl-Ph | -CHOH-1-methylindol-3-yl |

TABLE 4 (cont'd)

| $X^1$ | r | $R^2$ | $R^3$ |
|---|---|---|---|
| $CF_3$ | 1 | p-Cl-Ph | $-NH(CH_2)_{1-3}$-3-indolyl |
| $CF_3$ | 1 | p-Cl-Ph | NHCO-p-halo-phenyl |
| $CF_3$ | 1 | p-Cl-Ph | NHCO-m-halo-phenyl |
| $CF_3$ | 1 | p-Cl-Ph | NHCO-2-benzofuranyl |
| $CF_3$ | 1 | p-Cl-Ph | 2-(3-methylindenyl) |
| $CF_3$ | 1 | p-Cl-Ph | phenylethenyl |
| $CF_3$ | 1 | p-Cl-Ph | NHCONH-p-halo-phenyl |
| $CF_3$ | 1 | Ph | $-CH_2$-2-indolyl |
| $CF_3$ | 1 | Ph | $-CH_2$-3-indolyl |
| $CF_3$ | 1 | Ph | $-NHCO(CH_2)_{0-2}$-2-indolyl |
| $CF_3$ | 1 | Ph | -CHOH-1-methylindol-3-yl |
| $CF_3$ | 1 | Ph | $-NH(CH_2)_{1-3}$-3-indolyl |
| $CF_3$ | 1 | Ph | NHCO-p-halo-phenyl |
| $CF_3$ | 1 | Ph | NHCO-m-halo-phenyl |

TABLE 4 (cont'd)

| $X^1$ | r | $R^2$ | $R^3$ |
|---|---|---|---|
| $CF_3$ | 1 | Ph | NHCO-2-benzofuranyl |
| $CF_3$ | 1 | Ph | 2-(3-methylindenyl) |
| $CF_3$ | 1 | Ph | phenylethenyl |
| $CF_3$ | 1 | Ph | NHCONH-p-halo-phenyl |
| $CF_3$ | 1 | o-F-Ph | $-CH_2$-2-indolyl |
| $CF_3$ | 1 | o-F-Ph | $-CH_2$-3-indolyl |
| $CF_3$ | 1 | o-F-Ph | $-NHCO(CH_2)_{0-2}$-2-indolyl |
| $CF_3$ | 1 | o-F-Ph | -CHOH-1-methylindol-3-yl |
| $CF_3$ | 1 | o-F-Ph | $-NH(CH_2)_{1-3}$-3-indolyl |
| $CF_3$ | 1 | o-F-Ph | NHCO-p-halo-phenyl |
| $CF_3$ | 1 | o-F-Ph | NHCO-m-halo-phenyl |
| $CF_3$ | 1 | o-F-Ph | NHCO-2-benzofuranyl |
| $CF_3$ | 1 | o-F-Ph | 2-(3-methylindenyl) |
| $CF_3$ | 1 | o-F-Ph | phenylethenyl |

TABLE 4 (cont'd)

| $X^1$ | r | $R^2$ | $R^3$ |
|---|---|---|---|
| $CF_3$ | 1 | o-F-Ph | NHCONH-p-halo-phenyl |
| $CF_3$ | 1 | p-F-Ph | $-CH_2$-2-indolyl |
| $CF_3$ | 1 | p-F-Ph | $-CH_2$-3-indolyl |
| $CF_3$ | 1 | p-F-Ph | $-NHCO(CH_2)_{0-2}$-2-indolyl |
| $CF_3$ | 1 | p-F-Ph | -CHOH-1-methylindol-3-yl |
| $CF_3$ | 1 | p-F-Ph | $-NH(CH_2)_{1-3}$-3-indolyl |
| $CF_3$ | 1 | p-F-Ph | NHCO-p-halo-phenyl |
| $CF_3$ | 1 | p-F-Ph | NHCO-m-halo-phenyl |
| $CF_3$ | 1 | p-F-Ph | NHCO-2-benzofuranyl |
| $CF_3$ | 1 | p-F-Ph | 2-(3-methylindenyl) |
| $CF_3$ | 1 | p-F-Ph | phenylethenyl |
| $CF_3$ | 1 | p-F-Ph | NHCONH-p-halo-phenyl |
| $NO_2$ | 1 | p-Cl-Ph | $-CH_2$-2-indolyl |
| $NO_2$ | 1 | p-Cl-Ph | $-CH_2$-3-indolyl |

TABLE 4 (cont'd)

| $X^1$ | r | $R^2$ | $R^3$ |
|---|---|---|---|
| $NO_2$ | 1 | p-Cl-Ph | $-NHCO(CH_2)_{0-2}$-2-indolyl |
| $NO_2$ | 1 | p-Cl-Ph | -CHOH-1-methylindol-3-yl |
| $NO_2$ | 1 | p-Cl-Ph | $-NH(CH_2)_{1-3}$-3-indolyl |
| $NO_2$ | 1 | p-Cl-Ph | NHCO-p-halo-phenyl |
| $NO_2$ | 1 | p-Cl-Ph | NHCO-m-halo-phenyl |
| $NO_2$ | 1 | p-Cl-Ph | NHCO-2-benzofuranyl |
| $NO_2$ | 1 | p-Cl-Ph | 2-(3-methylindenyl) |
| $NO_2$ | 1 | p-Cl-Ph | phenylethenyl |
| $NO_2$ | 1 | p-Cl-Ph | NHCONH-p-halo-phenyl |
| $NO_2$ | 1 | Ph | $-CH_2$-2-indolyl |
| $NO_2$ | 1 | Ph | $-CH_2$-3-indolyl |
| $NO_2$ | 1 | Ph | $-NHCO(CH_2)_{0-2}$-2-indolyl |
| $NO_2$ | 1 | Ph | -CHOH-1-methylindol-3-yl |
| $NO_2$ | 1 | Ph | $-NH(CH_2)_{1-3}$-3-indolyl |

TABLE 4 (cont'd)

| $X^1$ | r | $R^2$ | $R^3$ |
|---|---|---|---|
| $NO_2$ | 1 | Ph | NHCO-p-halo-phenyl |
| $NO_2$ | 1 | Ph | NHCO-m-halo-phenyl |
| $NO_2$ | 1 | Ph | NHCO-2-benzofuranyl |
| $NO_2$ | 1 | Ph | 2-(3-methylindenyl) |
| $NO_2$ | 1 | Ph | phenylethenyl |
| $NO_2$ | 1 | Ph | NHCONH-p-halo-phenyl |
| $NO_2$ | 1 | o-F-Ph | $-CH_2$-2-indolyl |
| $NO_2$ | 1 | o-F-Ph | $-CH_2$-3-indolyl |
| $NO_2$ | 1 | o-F-Ph | $-NHCO(CH_2)_{0-2}$-2-indolyl |
| $NO_2$ | 1 | o-F-Ph | -CHOH-1-methylindol-3-yl |
| $NO_2$ | 1 | o-F-Ph | $-NH(CH_2)_{1-3}$-3-indolyl |
| $NO_2$ | 1 | o-F-Ph | NHCO-p-halo-phenyl |
| $NO_2$ | 1 | c-F-Ph | NHCO-m-halo-phenyl |
| $NO_2$ | 1 | o-F-Ph | NHCO-2-benzofuranyl |

TABLE 4 (cont'd)

| $X^1$ | r | $R^2$ | $R^3$ |
|---|---|---|---|
| $NO_2$ | 1 | o-F-Ph | 2-(3-methylindenyl) |
| $NO_2$ | 1 | o-F-Ph | phenylethenyl |
| $NO_2$ | 1 | o-F-Ph | NHCONH-p-halo-phenyl |
| $NO_2$ | 1 | p-F-Ph | $-CH_2$-2-indolyl |
| $NO_2$ | 1 | p-F-Ph | $-CH_2$-3-indolyl |
| $NO_2$ | 1 | p-F-Ph | $-NHCO(CH_2)_{0-2}$-2-indolyl |
| $NO_2$ | 1 | p-F-Ph | -CHOH-1-methylindol-3-yl |
| $NO_2$ | 1 | p-F-Ph | $-NH(CH_2)_{1-3}$-3-indolyl |
| $NO_2$ | 1 | p-F-Ph | NHCO-p-halo-phenyl |
| $NO_2$ | 1 | p-F-Ph | NHCO-m-halo-phenyl |
| $NO_2$ | 1 | p-F-Ph | NHCO-2-benzofuranyl |
| $NO_2$ | 1 | p-F-Ph | 2-(3-methylindenyl) |
| $NO_2$ | 1 | p-F-Ph | phenylethenyl |
| $NO_2$ | 1 | p-F-Ph | NHCONH-p-halo-phenyl |

TABLE 4 (cont'd)

| $X^1$ | r | $R^2$ | $R^3$ |
|---|---|---|---|
| $CH_3$ | 1 | phenyl | $-CH_2$-2-indolyl |
| $CH_3$ | 1 | phenyl | $-CH_2$-3-indolyl |
| $CH_3$ | 1 | phenyl | $-NHCO(CH_2)_{0-2}$-2-indolyl |
| $CH_3$ | 1 | phenyl | -CHOH-1-methylindol-3-yl |
| $CH_3$ | 1 | phenyl | $-NH(CH_2)_{1-3}$-3-indolyl |
| $CH_3$ | 1 | phenyl | NHCO-p-halo-phenyl |
| $CH_3$ | 1 | phenyl | NHCO-m-halo-phenyl |
| $CH_3$ | 1 | phenyl | NHCO-2-benzofuranyl |
| $CH_3$ | 1 | phenyl | 2-(3-methylindenyl) |
| $CH_3$ | 1 | phenyl | phenylethenyl |
| $CH_3$ | 1 | phenyl | NHCONH-p-halo-phenyl |
| H | 1 | o-F-Ph | $-CH_2$-2-indolyl |
| H | 1 | o-F-Ph | $-CH_2$-3-indolyl |
| H | 1 | o-F-Ph | $-NHCO(CH_2)_{0-2}$-2-indolyl |

TABLE 4 (cont'd)

| $X^1$ | r | $R^2$ | $R^3$ |
|---|---|---|---|
| H | 1 | o-F-Ph | -CHOH-1-methylindol-3-yl |
| H | 1 | o-F-Ph | $-NH(CH_2)_{1-3}$-3-indolyl |
| H | 1 | o-F-Ph | NHCO-p-halo-phenyl |
| H | 1 | o-F-Ph | NHCO-m-halo-phenyl |
| H | 1 | o-F-Ph | NHCO-2-benzofuranyl |
| H | 1 | o-F-Ph | 2-(3-methylindenyl) |
| H | 1 | o-F-Ph | phenylethenyl |
| H | 1 | o-F-Ph | NHCONH-p-halo-phenyl |
| H | 1 | p-Cl-Ph | $-CH_2$-2-indolyl |
| H | 1 | p-Cl-Ph | $-CH_2$-3-indolyl |
| H | 1 | p-Cl-Ph | $-NHCO(CH_2)_{0-2}$-2-indolyl |
| H | 1 | p-Cl-Ph | -CHOH-1-methylindol-3-yl |
| H | 1 | p-Cl-Ph | $-NH(CH_2)_{1-3}$-3-indolyl |
| H | 1 | P-Cl-Ph | NHCO-p-halo-phenyl |

TABLE 4 (cont'd)

| $X^1$ | r | $R^2$ | $R^3$ |
|---|---|---|---|
| H | 1 | P-Cl-Ph | NHCO-m-halo-phenyl |
| H | 1 | P-Cl-Ph | NHCO-2-benzofuranyl |
| H | 1 | P-Cl-Ph | 2-(3-methylindenyl) |
| H | 1 | P-Cl-Ph | phenylethenyl |
| H | 1 | P-Cl-Ph | NHCONH-p-halo-phenyl |
| H | 1 | Ph | -CO-3-thiophene |
| H | 1 | o-F-Ph | -CO-3-thiophene |
| H | 1 | p-Cl-Ph | -CO-3-thiophene |
| H | 1 | phenyl | -CO-3-thiophene |
| H | 1 | p-F-Ph | -CO-3-thiophene |
| H | 1 | 2,4-di-Cl-Ph | -CO-3-thiophene |
| H | 1 | 2,6-di-F-Ph | -CO-3-thiophene |
| H | 1 | $-CH_2COO$-t-butyl | -CO-3-thiophene |
| H | 1 | $-CH_2COOEt$ | -CO-3-thiophene |

TABLE 4 (cont'd)

| $X^1$ | r | $R^2$ | $R^3$ |
|---|---|---|---|
| H | 1 | o-F-Ph | $CH_2$-2 or 3-(1-methyl indolyl) |
| H | 1 | $-CH_2COO$-t-butyl | $CH_2$-2 or 3-(1-methyl indolyl) |
| H | 1 | $-CH_2COOEt$ | $CH_2$-2 or 3-(1-methyl indolyl) |
| H | 1 | Ph | $CH_2$-2 or 3-(1-methyl indolyl) |
| H | 1 | o-F-Ph | $NHCO(CH_2)_{0-2}$-2-indolyl |
| H | 1 | $-CH_2COO$-t-butyl | $NHCO(CH_2)_{0-2}$-2-indolyl |
| H | 1 | $-CH_2COOEt$ | $NHCO(CH_2)_{0-2}$-2-indolyl |
| H | 1 | Ph | $NHCO(CH_2)_{0-2}$-2-indolyl |
| H | 1 | o-F-Ph | $NHCO(CH_2)_{0-2}$-2 or 3-(1-methylindolyl) |
| H | 1 | $-CH_2COO$-t-butyl | $NHCO(CH_2)_{0-2}$-2 or 3-(1-methylindolyl) |

TABLE 4 (cont'd)

| $X^1$ | r | $R^2$ | $R^3$ |
|---|---|---|---|
| H | 1 | $-CH_2COOEt$ | $NHCO(CH_2)_{0-2}$-2 or 3-(1-methylindolyl) |
| H | 1 | Ph | $NHCO(CH_2)_{0-2}$-2 or 3-(1-methylindolyl) |
| H | 1 | o-F-Ph | Boc-Trp-NH |
| H | 1 | $-CH_2COO$-t-butyl | Boc-Trp-NH |
| H | 1 | $-CH_2COOEt$ | Boc-Trp-NH |
| H | 1 | Ph | Boc-Trp-NH |
| H | 1 | o-F-Ph | Cbz-Trp-NH |
| H | 1 | $-CH_2COO$-t-butyl | Cbz-Trp-NH |
| H | 1 | $-CH_2COOEt$ | Cbz-Trp-NH |
| H | 1 | Ph | Cbz-Trp-NH |
| H | 1 | o-F-Ph | $NH(CH_2)_{1-3}$-2-indolyl |
| H | 1 | $-CH_2COO$-t-butyl | $NH(CH_2)_{1-3}$-2-indolyl |
| H | 1 | $-CH_2COOEt$ | $NH(CH_2)_{1-3}$-2-indolyl |

TABLE 4 (cont'd)

| $X^1$ | r | $R^2$ | $R^3$ |
|---|---|---|---|
| H | 1 | Ph | $NH(CH_2)_{1-3}$-2-indolyl |
| H | 1 | o-F-Ph | $NH(CH_2)_{1-3}$-2 or 3-(1-methylindolyl) |
| H | 1 | $-CH_2COO$-t-butyl | $NH(CH_2)_{1-3}$-2 or 3-(1-methylindolyl) |
| H | 1 | $-CH_2COOEt$ | $NH(CH_2)_{1-3}$-2 or 3-(1-methylindolyl) |
| H | 1 | Ph | $NH(CH_2)_{1-3}$-2 or 3-(1-methylindolyl) |
| H | 1 | o-F-Ph | CHOH-2 or 3-indolyl |
| H | 1 | $-CH_2COO$-t-butyl | CHOH-2 or 3-indolyl |
| H | 1 | $-CH_2COOEt$ | CHOH-2 or 3-indolyl |
| H | 1 | Ph | CHOH-2 or 3-indolyl |
| H | 1 | o-F-Ph | CHOH-2-(1-methylindolyl) |
| H | 1 | $-CH_2COO$-t-butyl | CHOH-2-(1-methylindolyl) |
| H | 1 | $-CH_2COOEt$ | CHOH-2-(1-methylindolyl) |

TABLE 4 (cont'd)

| $X^1$ | r | $R^2$ | $R^3$ |
|---|---|---|---|
| H | 1 | Ph | CHOH-2-(1-methylindolyl) |
| H | 1 | $-CH_2COO$-t-butyl | CO-2-thiophene |
| H | 1 | $-CH_2COOEt$ | CO-2-thiophene |
| H | 1 | Ph | CO-2-thiophene |
| H | 1 | o-F-Ph | $NHCH(CH_2)_2$-2-indolyl<br>COOEt |
| H | 1 | $-CH_2COO$-t-butyl | $NHCH(CH_2)_2$-2-indolyl<br>COOEt |
| H | 1 | $-CH_2COOEt$ | $NHCH(CH_2)_2$-2-indolyl<br>COOEt |
| H | 1 | Ph | $NHCH(CH_2)_2$-2-indolyl<br>COOEt |
| H | 1 | o-F-Ph | COOEt<br>$NHCH(CH_2)_2$-2 or 3-(1-methyl-indolyl) |
| H | 1 | $-CH_2COO$-t-butyl | COOEt<br>$NHCH(CH_2)_2$-2 or 3-(1-methyl-indolyl) |

TABLE 4 (cont'd)

| $X^1$ | r | $R^2$ | $R^3$ |
|---|---|---|---|
| H | 1 | $-CH_2COOEt$ | COOEt<br>$NHCH(CH_2)_2$-2 or 3-(1-methyl-indolyl) |
| H | 1 | Ph | COOEt<br>$NHCH(CH_2)_2$-2 or 3-(1-methyl-indolyl) |
| H | 1 | o-F-Ph | $NHCH(CH_2)_2$-2 or 3-indolyl<br>COO-t-Butyl |
| H | 1 | $-CH_2$COO-t-butyl | $NHCH(CH_2)_2$-2 or 3-indolyl<br>COO-t-Butyl |
| H | 1 | $-CH_2COOEt$ | $NHCH(CH_2)_2$-2 or 3-indolyl<br>COO-t-Butyl |
| H | 1 | Ph | $NHCH(CH_2)_2$-2 or 3-indolyl<br>COO-t-Butyl |
| H | 1 | o-F-Ph | COO-t-Butyl<br>$NHCH(CH_2)_2$-2 or 3-(1-methyl-indolyl) |
| H | 1 | $-CH_2$COO-t-butyl | COO-t-Butyl<br>$NHCH(CH_2)_2$-2 or 3-(1-methyl-indolyl) |

TABLE 4 (cont'd)

| $X^1$ | r | $R^2$ | $R^3$ |
|---|---|---|---|
| H | 1 | $-CH_2COOEt$ | $NHCH(COO\text{-}t\text{-}Butyl)(CH_2)_2$-2 or 3-(1-methylindolyl) |
| H | 1 | Ph | $NHCH(COO\text{-}t\text{-}Butyl)(CH_2)_2$-2 or 3-(1-methylindolyl) |

The invention is further defined by reference to the following preparations and examples, which are intended to be illustrative and not limiting.

All temperatures are in degrees Celsius.

Preparation 1

1,3-Dihydro-3(R)-(3'-indolyl)methyl-5-(2'-fluorophenyl)-2H-1,4-benzodiazepin-2-thione

1,3-Dihydro-3(R)-(3'-indolyl)methyl-5-(2'-fluorophenyl)-2H-1,4-benzodiazepin-2-one (6.98 g, 18.20 mmole) was refluxed with 4.41 g (10.92 mmole) of 2,4-bis-(4-methoxyphenyl)-2,4-dithioxo-1,3,2,4-dithiadiphosphetane in 100 ml of toluene for 1.5 hours. The solvent was removed *in vacuo* and the residue partitioned between ethyl acetate and 10% sodium hydroxide solution. The organic phase was washed with 10% sodium hydroxide (3 x 50 ml) and brine, then dried ($MgSO_4$) and rotoevaporated to give an orange oil (10 g). Plug filtration of the crude product through silica gel (100 g) afforded a solid which was recrystallized from ether to afford the analytical sample as an ether solvate.
m.p. 147-148°C.
Pmr confirmed the structure of the title compound.

EXAMPLE 1

2-Phenylamino-3(R)-(3'-indolyl)methyl-5-(2'-fluorophenyl)-2H-1,4-benzodiazepine

2-Methylthio-3(R)-(3'-indolyl)methyl-5-(2'-fluorophenyl)-2H-1,4-benzodiazepine (170 mg, 0.42 mmole) was mixed with 138 µl (3.6 equivalents) of

aniline and the whole immersed in a preheated bath at 80°C. After 2 days, the dark reaction mixture was chromatographed on silica gel (hexane-ethyl acetate elution 2:1 v/v) to give the analytical sample as an off-white solid, m.p. 127°.

TLC, HPLC: greater than 98% pure

MS (20 ev): 458 ($M^+$), 366, 329, 130.

Pmr ($CDCl_3$): Confirmed the structure of the title compound.

Elemental Analysis: $C_{30}H_{23}FN_4$ $0.2H_2O$

Calc'd: N, 12.12; C, 77.96; H, 5.10.

Found: N, 12.22; C, 77.89; H, 4.93.

## EXAMPLE 2

**2-Methylthio-3(R)-(3'-indolyl)methyl-5-(2'-fluorophenyl)2H-1,4-benzodiazepine**

Thioamide according to Preparation 1 (2.66 g, 6.66 mmole) was added to a suspension of 40% sodium hydroxide solution (30 ml), 50 ml of toluene, 25 ml of tetrahydrofuran, and 10 ml of water. The reaction mixture was then treated with 1.49 g (0.66 equivalents) of tetra-*n*-butyl ammonium sulfate. After 5 minutes, 456 µl (1.1 equivalents) of iodomethane was added to the rapidly stirred suspension. The reaction was stirred at room temperature for 15 minutes more, poured into a separatory funnel and the phases separated. The organic phase was washed with water and brine, then dried ($MgSO_4$) and concentrated to yield 2.8 g of the crude product. The analytical sample was obtained *via* flash column chromatography on silica gel (hexane-ethyl acetate elution, 2:1 v/v).

TLC, HPLC: greater than 97.9% pure

MS (20 ev): 413 ($M^+$), 284, 130.
Pmr ($CDCl_3$): Spectrum according to theory. S$\underline{CH}_3$ (2.49 ppm)
Elemental Analysis: $C_{25}H_{20}FN_3OS$ $0.3H_2O$
Calc'd: N, 10.03; C, 71.67; H, 4.95.
Found: N, 9.84; C, 71.74; H, 4.66.

## EXAMPLE 3

2-Amino-3(R)-(3'-indolyl)methyl-5-(2'-fluorophenyl)-2H-1,4-benzodiazepine

Thioamide according to Preparation 1 (170 mg, 0.43 mmole) was dissolved in warm methanol (6 ml). Concentrated ammonium hydroxide solution (58%) (2 ml) was added followed by 117 mg (0.43 mmole) of mercuric chloride. The reaction mixture was stirred vigorously for 3 hours, cooled and filtered through Celite. The filtrate was concentrated to dryness and the resulting white solid (200 mg) chromatographed on silica gel (chloroform-ethanol-ammonia, 90:10:1 v/v) to give the analytical product (120 mg).

TLC, HPLC: greater than 98% pure
MS (20 ev): 382 ($M^+$), 253, 211, 130.
Pmr ($CDCl_3$): Confirmed the structure of the title compound.
$^{19}F$ nmr ($CD_3OD$): 113 ppm
Elemental Analysis: $C_{24}H_{19}FN_4$ $0.25H_2O$
Calc'd: N, 14.48; C, 74.49; H, 5.07.
Found: N, 14.72; C, 74.41; H, 4.92.

EXAMPLE 4

2-Methylamino-3(R)-(3'-indolyl)methyl-5-(2'-fluorophenyl)-2H-1,4-benzodiazepine

This compound was prepared according to Example 3 using 2 g (5 mmole) of thioamide according to Preparation 1, 2 g (7.4 mmole) of mercuric chloride, and 20 equivalents of methylamine in 30 ml of dry tetrahydrofuran. Yield of crude product = 2.05 g as a white powder. TLC, HPLC: greater than 95% pure

MS (14 ev): 396 ($M^+$), 277, 211.

Pmr ($CDCl_3$): Confirmed the structure of the title compound; N-methyl protons resonate as a doublet at 2.94 ppm.

Elemental Analysis: $C_{25}H_{21}FN_4$ $0.2CHCl_3$

Calc'd: N, 13.33; C, 72.00; H, 5.08.

Found: N, 13.11; C, 72.08; H, 5.26.

EXAMPLE 5

2-Ethoxycarbonylmethylamino-3(R)-(3'-indolyl)methyl-5-(2'-fluorophenyl)-2H-1,4-benzodiazepine

This compound was prepared according to Example 3 using 300 mg (0.75 mmole) of thioamide according to Preparation 1, 310 mg (1.13 mmole) of mercuric chloride and four equivalents of glycine ethyl ester in 25 ml of dry tetrahydrofuran. Work-up afforded 400 mg of crude product. The analytical sample was obtained *via* silica gel chromatography (chloroform-methanol 99:1 v/v).

HPLC, TLC: greater than 92% pure.
Pmr ($CDCl_3$): Confirmed the structure of the title compound.
MS (14 ev): 468 ($M^+$), 422, 339.
Elemental Analysis: $C_{28}H_{25}FN_4O_2$ 0.1 $CHCl_3$
Calc'd: N, 11.66; C, 70.24; H, 5.26.
Found: N, 11.39; C, 70.46; H, 5.43.

## EXAMPLE 6

2-Cyanoamino-3(R)-(3'-indolyl)methyl-5-(2'-fluorophenyl)-2H-1,4-benzodiazepine

This compound was prepared according to Example 3 using 300 mg (0.75 mmole) of thioamide according to Preparation 1, 310 mg (1.2 mmole) of mercuric chloride and 3 equivalents of cyanamide in 20 ml of dry tetrahydrofuran. Yield of crude product was 330 mg. Chromatography on silica gel using 1% methanol in chloroform gave the analytical sample.
TLC, HPLC: greater than 96% pure.
MS (14 ev): 407 ($M^+$), 378, 278.
Pmr ($CDCl_3$): Confirmed the structure of the title compound.
Elemental Analysis: $C_{25}H_{18}FN_5$ $1.05CHCl_3$.
Calc'd: N, 13.14; C, 58.71; H, 3.60.
Found: N, 13.11; C, 58.59; H, 3.66.

## EXAMPLE 7

2-Propylamino-3(R)-(3'-indolyl)methyl-5-(2-fluorophenyl)-2H-1,4-benzodiazepine

1,3-Dihydro-3(R)-(3'-indolyl)methyl-5-(2-fluorophenyl)-2H-1,4-benzodiazepin-2-thione (350 mg, 0.88 mmole) was dissolved in 20 ml of dry methanol.

The solution was warmed to 50°C and treated in succession with n-propylamine (1 ml, 12.2 mmole) and mercuric chloride (357 mg, 1.31 mmole). The reaction mixture was stirred at 50°C for two hours during the course of which a black precipitate was deposited. The reaction mixture was filtered, concentrated *in vacuo* and the residue dissolved in ethyl acetate. The organic phase was washed with sodium thiosulfate solution (2x30 ml) and brine, then dried ($MgSO_4$) and concentrated to yield 400 mg of crude product. Silica gel chromatography of the crude product (hexane-acetone elution, 3:1 v/v) afforded the analytical sample.

TLC, HPLC: greater than 99.5% pure.

Pmr ($CDCl_3$): according to theory.

MS (14 ev): 424 ($M^+$).

Elemental Analysis: $C_{27}H_{25}FN_4 \cdot 0.2H_2O$:

Calc'd: N, 13.08; C, 75.74; H, 5.98.

Found: N, 12.56; C, 75.93; H, 6.26.

EXAMPLE 8

2-Carboxymethylamino-3(R)-(3'-indolyl)methyl-5-(2'-fluorophenyl)-2H-1,4-benzodiazepine

To a solution of 1,3-dihydro-3(R)-(3'-indolyl)methyl-5-(2-fluorophenyl)-2H-1,4-benzodiazepin-2-thione (300 mg, 0.75 mmole) in 15 ml of dry tetrahydrofuran was added mercuric chloride (310 mg, 1.13 mmole) and glycine *tert*-butyl ester hydrochloride. The pH of the resulting suspension was adjusted to 8.5 with triethylamine and the reaction mixture was then heated at 55°C for 2 hours. The reaction was cooled, filtered through a Celite pad

and concentrated. The residual semi-solid was dissolved in ethyl acetate (150 ml) and washed with sodium thiosulfate solution (2x50 ml) and brine. The dried (($MgSO_4$) organic phase was concentrated to yield 500 mg of crude product which was flash chromatographed on silica gel (hexane-ethyl acetate elution, 1:1 v/v) to remove polar biproducts. A portion of the purified intermediate ester (220 mg) was dissolved in 50 ml of ethyl acetate, cooled to 0°C and treated with a continuous stream of hydrogen chloride gas for 30 minutes. The reaction mixture was then allowed to warm to room temperature and stand for 5 hours. Excess reagent and solvent were removed in vacuo to yield 200 mg of an orange-brown powder. The analytical material was obtained via silica gel chromatography (chloroform-methanol-acetic acid elution, 90:10:1 then 85:15:1.5 v/v) as an off-white powder (120 mg).

HPLC: greater than 96% pure.

Pmr ($CD_3OD$): according to theory.

MS (14 ev): 422 ($M^+-H_2O$), 293, 277, 264, 246.

Elemental Analysis: $C_{26}H_{21}FN_4O_2 \cdot 0.8CHCl_3 \cdot 0.8HOAc$:

Calc'd: N, 9.59; C, 58.40; H, 4.31.

Found: N, 9.50; C, 58.31; H, 4.49.

Claims to the invention follow.

WHAT IS CLAIMED IS:

1. A compound of Formula I:

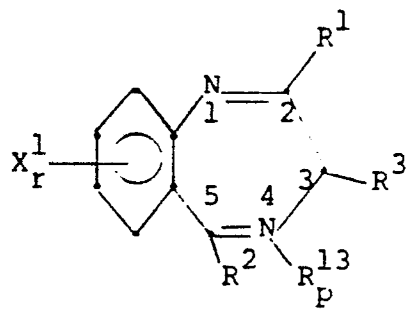

I

wherein

$R^1$ is $-NR^{16}R^{17}$, $-SR^{11}$, or $-OR^{11}$;

$R^2$ is H, loweralkyl, substituted or unsubstituted phenyl wherein the substituents may be 1 or 2 of halo, loweralkyl, loweralkoxy, loweralkylthio, carboxyl, carboxyloweralkyl, nitro, $-CF_3$, $\overset{O}{\overset{\|}{OC}}R^4$ or hydroxy, or $-(CH_2)_mCOOR^6$;

$R^3$ is $-(CH_2)_nR^7$, $-(CH_2)_n\overset{OH}{\overset{|}{C}}HR^7$, $-(CH_2)_nX^9\overset{O}{\overset{\|}{C}}\underset{NHCOOR^{14}}{\underset{|}{C}}HCH_2R^7$,

$-(CH_2)_n\overset{O}{\overset{\|}{C}}R^7$, $-(CH_2)_nNR^{18}(CH_2)_qR^7$,

$-(CH_2)_nNR^{18}\overset{\overset{R^7}{|}}{\overset{(CH_2)_q}{\overset{|}{C}}}HCOOR^6$, $-(CH_2)_nX^9\overset{O}{\overset{\|}{C}}(CH_2)_qR^7$,

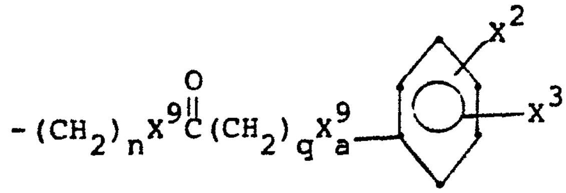,

$-(CH_2)_nNR^{18}SO_2(CH_2)_qR^7$, or

$-(CH_2)_n-X^9-\overset{O}{\overset{\|}{C}}-X^9_a-(CH_2)_n-R^7$;

$R^4$ and $R^5$ are, when separate, independently H, loweralkyl, or cycloloweralkyl or when joined form, with the N, a heterocycle $-N\overset{\frown}{\quad}(CH_2)_{n'}$, where n' is 2-6;

$R^6$ is H, loweralkyl, cycloloweralkyl, substituted or unsubstituted phenyl, or substituted or unsubstituted phenylloweralkyl (wherein the substituents may be 1 or 2 of halo, loweralkyl, loweralkoxy, nitro, or $CF_3$);

$R^7$ is H, α- or ß-naphthyl, substituted or unsubstituted phenyl (wherein the substituents may be 1 to 2 of halo, $-NO_2$, -OH, $-NR^4R^5$, loweralkyl, or loweralkoxy, $CF_3$, S-loweralkyl, cyano, phenyl, acetylamino, acetoxy, $SCF_3$, C≡CH, $CH_2SCF_3$, $OCHF_2$, SH, S-phenyl, or $PO_3H$);

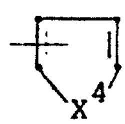, 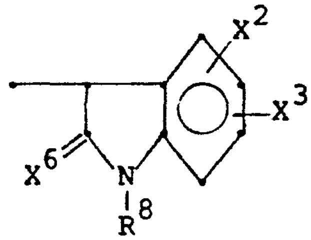, 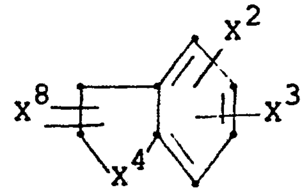,

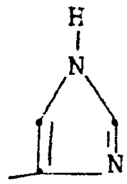 , 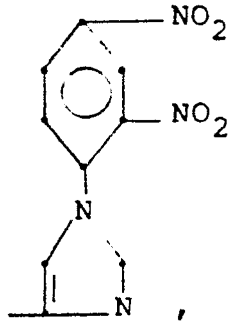 ,

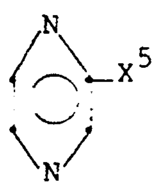 , 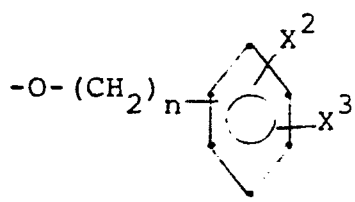 ,

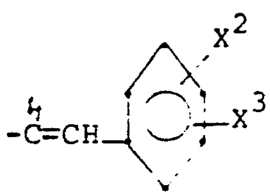 , 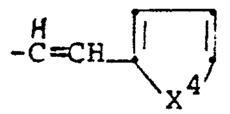 , 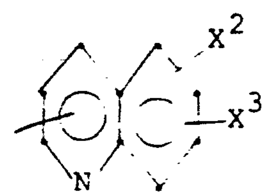 ,

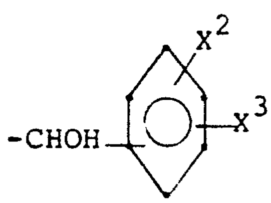 , or 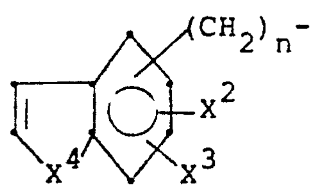

(with the provisos that q is not 0 or 1 in $-(CH_2)_nNH(CH_2)_qR^7$ and that q is not 0 in

$-(CH_2)_nNR^{18}\overset{\overset{\overset{R^7}{|}}{(CH_2)_q}}{\overset{|}{C}}HCOOR^6$ when $R^7$ is $-O-(CH_2)_n-$ 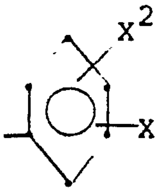 )

$R^8$ is H, loweralkyl, cycloloweralkyl, $-(CH_2)_mCONH_2$, $-(CH_2)_mCOOR^6$, $-(CH_2)_n$-cycloloweralkyl,

$-(CH_2)_mNR^4R^5$ , $-(CH_2)_m$ 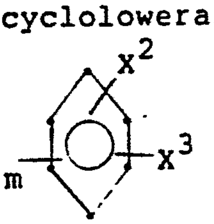 ,

$-(CH_2)_nCO(CH_2)_q$ 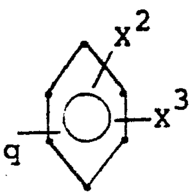 , or $-COCHNHCOOR^{11}$;
$CH_2R^{12}$

$R^{11}$ is loweralkyl or cycloloweralkyl;

$R^{12}$ is loweralkyl, cycloloweralkyl, or straight chain or branched hydroxyloweralkyl;

$R^{13}$ is O;

$R^{14}$ is loweralkyl or phenylloweralkyl;

$R^{16}$ and $R^{17}$ are, when separate, independently H, loweralkyl,

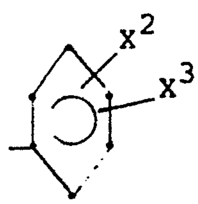, $-(CH_2)_mCOOR^6$, or -CN; or, when joined form, with N, a heterocycle 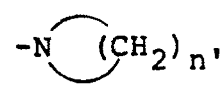 wherein n' is 2-6,

$R^{18}$ is H, loweralkyl, or acyl;

m is 1-4;

n is 0-4;

p is 0 or 1;

q is 0-4;

r is 1 or 2;

$X^1$ is H, $-NO_2$, $CF_3$ CN, OH, loweralkyl, halo, loweralkylthio, loweralkoxy, $-(CH_2)_nCOOR^6$, $-NR^4R^5$, or $\overset{O}{\overset{\|}{O C}}-R^4$;

$X^2$ and $X^3$ are independently H, -OH, $-NO_2$, halo, loweralkylthio, loweralkyl, loweralkoxy, or $\overset{O}{\overset{\|}{O C}}R^4$;

$X^4$ is S, O, $CH_2$ or $NR^8$;

$X^5$ is H, $CF_3$, CN, $COOR^6$, $NO_2$, or halo;

$X^6$ is O or HH;

$X^8$ is H or loweralkyl;

$X^9$ and $X^9_a$ are independently $NR^{18}$, O;

and salts and quaternary ammonium salts of the compounds of formula I.

2. A compound of Claim 1 wherein:

$R^1$ is $-NR^{16}R^{17}$ or $-SR^{11}$;

$R^2$ is substituted or unsubstituted phenyl (wherein the substituents may be 1 or 2 of halo, loweralkyl, loweralkoxy, carboxyl, carboxyloweralkyl, nitro, $-CF_3$, or hydroxy), or $-(CH_2)_mCOOR^6$;

$R^3$ is $-(CH_2)_nR^7$, $-(CH_2)_nNH\overset{O}{\overset{\|}{C}}\underset{NHCOOR^{14}}{\underset{|}{C}H}CH_2R^7$, $-(CH_2)_n\overset{OH}{\overset{|}{C}}HR^7$,

$-(CH_2)_n\overset{O}{\overset{\|}{C}}R^7$, $-(CH_2)_nNH(CH_2)_qR^7$, $-NH(CH_2)_{2-3}NHCOR^7$,

Dr.IM.-vd
18.6.1985

$NH(CH_2)_{2-3}NHR^7$, 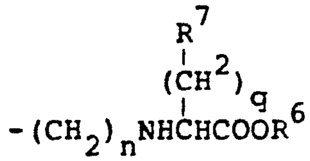,

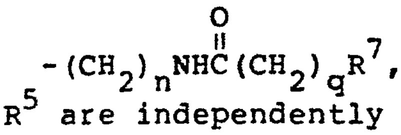, or 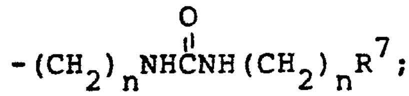;

$R^4$ and $R^5$ are independently H or loweralkyl;

$R^6$ is H or loweralkyl;

$R^7$ is α- or ß-naphthyl,

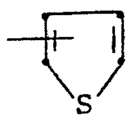, 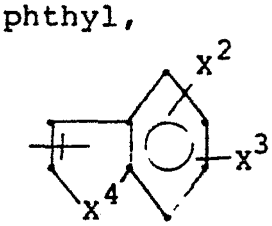, 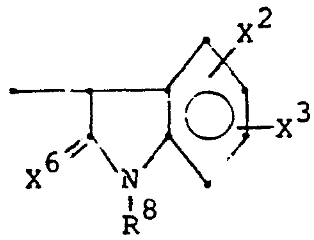

substituted or unsubstituted phenyl (wherein the substituents may be 1 to 2 of halo, $-NO_2$, -OH, $-NR^4R^5$, loweralkyl, $CF_3$, loweralkoxy, loweralkylthio, CN, C≡CH, $SCF_3$, 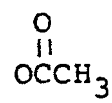, $OCHF_2$, or SPh), -CH=CH 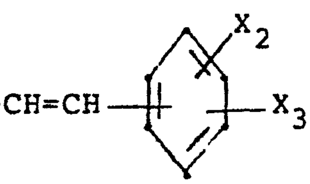, or -CH=CH 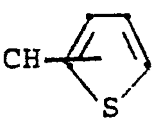;

$R^8$ is H, loweralkyl or

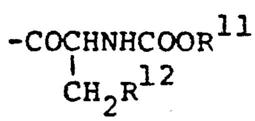 ;

$R^{11}$ and $R^{12}$ are independently loweralkyl;

$R^{14}$ is loweralkyl;

$R^{16}$ and $R^{17}$ are independently H, loweralkyl,

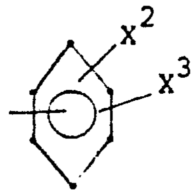, $-(CH_2)_mCOOR^6$, or -CN;

m is 1-4;
n is 0-4;
q is 0-4;
r is 1 or 2;
$X^1$ is H, $-NO_2$, $CF_3$ CN, OH, loweralkyl, halo, loweralkylthio, loweralkoxy, $-(CH_2)_nCOOR^6$, or $-NR^4R^5$;
$X^2$ and $X^3$ are independently H, -OH,$-NO_2$, halo, loweralkylthio, loweralkyl, or loweralkoxy;
$X^4$ is S, O, or $NR^8$;
$X^6$ is O or HH;

and salts and quaternary ammonium salts of said compounds.

3. A compound of Claim 2 wherein:

$R^1$ is $-NR^{16}R^{17}$;
$R^2$ is substituted or unsubstituted phenyl (wherein the substituents may be 1 or 2 of halo or carboxyl) or $-(CH_2)_{1-2}COOR^6$;

$R^3$ is $-(CH_2)_nR^7$, $-(CH_2)_nNH\overset{\overset{O}{\|}}{C}\underset{\underset{NHCOOR^{14}}{|}}{C}HCH_2R^7$, $-(CH_2)_n\overset{\overset{OH}{|}}{C}HR^7$,

Dr.IM.-vd
18.6.1985

$-(CH_2)_n\overset{O}{\overset{\|}{C}}R^7$, $-(CH_2)_nNH(CH_2)_qR^7$, $-(CH_2)_nNH\underset{}{\overset{R^7-(CH_2)_q}{CH}}COOR^6$,

or $-(CH_2)_nNH\overset{O}{\overset{\|}{C}}(CH_2)_qR^7$;

$R^6$ is H or loweralkyl;

$R^7$ is α- or ß-naphthyl,

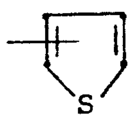,
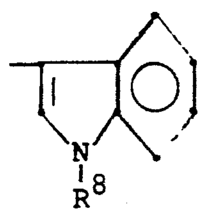,
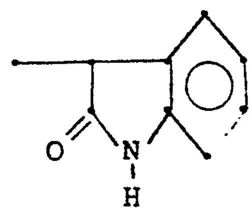,

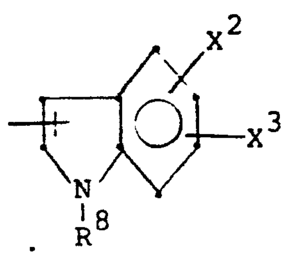,

substituted phenyl (wherein the substituents may be 1 or 2 of halo, loweralkyl, or $CF_3$),

or -CH=CH 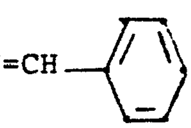

$R^8$ is H, methyl, or ethyl;

$R^{14}$ is t-butyl;

$R^{16}$ and $R^{17}$ are independently H, loweralkyl, or -CN;

n is 0-4;

q is 0-4;

r is 1 or 2;

$X^1$ is H, $-NO_2$, $CF_3$ CN, OH, or halo;

$X^2$ and $X^3$ are independently H, -OH, $-NO_2$, or halo;

and salts and quaternary ammonium salts of said compounds.

4. A compound of Claim 3 wherein:

$R^1$ is $-NH_2$, $-NH(CH_2)_{0-2}CH_3$, $NHCH_2COOH$, or -NHCN;

$R^2$ is phenyl, o-fluorophenyl, o-chlorophenyl, p-fluorophenyl, p-chlorophenyl, 2,6-difluorophenyl, $-CH_2COOEt$, $-CH_2COO$-t-Bu, o-carboxyphenyl, $-CH_2CH_2COOEt$, or $-CH_2CH_2COO$t-Bu;

$R^3$ is $-(CH_2)_{1-2}R^7$, $-(CH_2)_{0-1}NH\overset{O}{\overset{\|}{C}}\underset{NHCOOR^{14}}{\underset{|}{C}}HCH_2R^7$, $-\overset{OH}{\overset{|}{C}}HR^7$, $-\overset{O}{\overset{\|}{C}}R^7$,

$-(CH_2)_{0-1}NH(CH_2)_{1-2}R^7$,

$-(CH_2)_{0-1}NH\overset{R^7}{\overset{|}{C}}HCOOR^6$, or $-(CH_2)_{0-1}NH\overset{O}{\overset{\|}{C}}(CH_2)_{0-2}R^7$;

and the stereochemistry relates to D-tryptophan;

$R^6$ is H, methyl or ethyl;

$R^7$ is α- or ß-naphthyl, mono- or dihalophenyl,

Dr.IM.-vd
18.6.1985

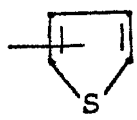 , 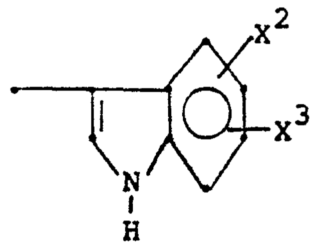 ,

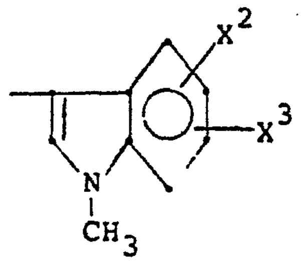 ,

$R^{14}$ is t-butyl;
r is 1;
$X^1$ is H, chloro, fluoro, or nitro;
$X^2$ and $X^3$ are independently H, -OH, fluoro or chloro;
and salts and quaternary ammonium salts thereof.

5. A compound of Claim 1 which is
2-Phenylamino-3(R)-(3'-indolyl)methyl-5-(2'-fluorophenyl)-2H-1,4-benzodiazepine;

2-Methylthio-3(R)-(3'-indolyl)methyl-5-(2'-fluorophenyl)2H-1,4-benzodiazepine;

2-Amino-3(R)-(3'-indolyl)methyl-5-(2'-fluorophenyl)-2H-1,4-benzodiazepine;

2-Methylamino-3(R)-(3'-indolyl)methyl-5-(2'-fluorophenyl)-2H-1,4-benzodiazepine;

Dr.IM.-vd
18.6.1985

2-Ethoxycarbonylmethylamino-3(R)-(3'-indolyl)methyl-5-(2'-fluorophenyl)-2H-1,4-benzodiazepine;

2-Cyanoamino-3(R)-(3'-indolyl)methyl-5-(2'-fluorophenyl)-2H-1,4-benzodiazepine;

2-n-Propylamino-3(R)-(3'-indolyl)methyl-5-(2'-fluorophenyl)-2H-1,4-benzodiazepine;

2-Carboxymethylamino-3(R)-(3'-indolyl)methyl-5-(2'-fluorophenyl)-2H-1,4-benzodiazepine.

6. A pharmaceutical composition useful for treating gastrointestinal disorders, central nervous system disorders, or regulating appetite in mammals, characterized in that it contains as pharmaceutically active ingredient at least one compound of formula I according to patent claim 1 or a pharmaceutically acceptable salt or pharmaceutically acceptable quaternary ammonium salt of the compound of formula I.

7. A pharmaceutical composition according to claim 6 characterized in that it contains as active ingredient at least one compound of formula I wherein:

$R^1$ is $-NR^{16}R^{17}$ or $-SR^{11}$;

$R^2$ is substituted or unsubstituted phenyl (wherein the substituents may be 1 or 2 of halo, loweralkyl, loweralkoxy, carboxyl, carboxyloweralkyl, nitro, $-CF_3$, or hydroxy), or $-(CH_2)_mCOOR^6$;

$R^3$ is $-(CH_2)_nR^7$, $-(CH_2)_nNH\overset{O}{\overset{\|}{C}}\underset{NHCOOR^{14}}{\underset{|}{C}}HCH_2R^7$, $-(CH_2)_n\overset{OH}{\overset{|}{C}}HR^7$,

$-(CH_2)_n\overset{O}{\overset{\|}{C}}R^7$, $-(CH_2)_nNH(CH_2)_qR^7$, $-NH(CH_2)_nNHR^7$,

$-NH(CH_2)_nNHCOR^7$, $-(CH_2)_nNH\overset{R^7-(CH_2)_q}{\overset{|}{C}}HCOOR^6$,

$-(CH_2)_nNH\overset{O}{\overset{\|}{C}}(CH_2)_qR^7$, or $-(CH)_nNH\overset{O}{\overset{\|}{C}}NH(CH_2)_nR^7$;

$R^4$ and $R^5$ are independently H or loweralkyl;
$R^6$ is H or loweralkyl;
$R^7$ is H, α- or ß-naphthyl,

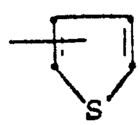 , 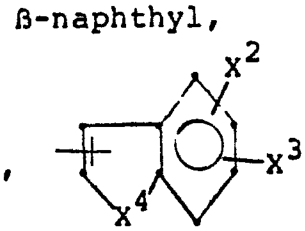 , 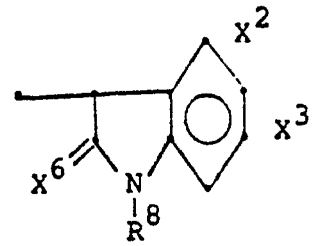

substituted or unsubstituted phenyl (wherein the substituents may be 1 to 2 of halo, $-NO_2$, -OH, $-NR^4R^5$, loweralkyl, loweralkoxy, $CF_3$, loweralkylthio, CN, C≡CH, $SCF_3$, $O\overset{O}{\overset{\|}{C}}CH_3$, $OCHF_2$, or SPh),

-CH=CH 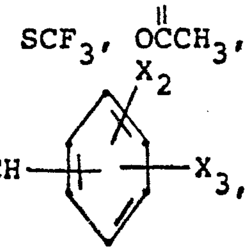 , or -CH=CH 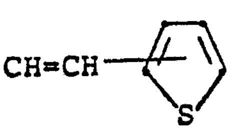 ;

$R^8$ is H, loweralkyl or

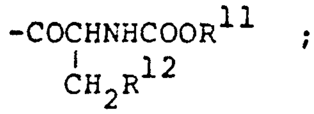

$R^{11}$ and $R^{12}$ are independently loweralkyl;
$R^{14}$ is loweralkyl;
$R^{16}$ and $R^{17}$ are independently H, loweralkyl,

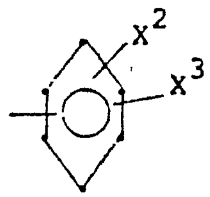 , $-(CH_2)_mCOOR^6$, or -CN;

m is 1-4;
n is 0-4;
q is 0-4;
r is 1 or 2;
$X^1$ is H, $-NO_2$, $CF_3$ CN, OH, loweralkyl, halo, loweralkylthio, loweralkoxy, $-(CH_2)_nCOOR^6$, or $-NR^4R^5$;
$X^2$ and $X^3$ are independently H, -OH, $-NO_2$, halo, loweralkylthio, loweralkyl, or loweralkoxy;
$X^4$ is S, O, or $NR^8$;
$X^6$ is O or HH;

or a pharmaceutically acceptable salt of the compounds of formula I or a pharmaceutically acceptable quaternary ammonium salt of the compounds of formula I.

8. Pharmaceutical composition according to claim 6 or 7 characterized in that it contains as

Dr.IM.-vd
18.6.1985

4

pharmaceutically active ingredient a compound of formula I according to one of the claims 3, 4 or 5.

9. A pharmaceutical composition according to one of the claims 6 - 8 characterized in that it contains as further component a pharmaceutically acceptable carrier.